# EUROPEAN PATENT SPECIFICATION

(11) **EP 4 552 617 B1**
(45) Date of publication and mention of the grant of the patent: **07.10.2026**
(21) Application number: 25166640.0
(22) Date of filing: 04.03.2022
(51) Int. Cl.: A61F 2/30, A61F 2/40, A61F 2/46

(54) **IMPLANT WITH SUTURE RETENTION STRUCTURE**
IMPLANTAT MIT NAHTHALTESTRUKTUR
IMPLANT AVEC STRUCTURE DE RÉTENTION DE SUTURE

(30) Priority: 04.03.2021 US 202163156437 P; 08.03.2021 US 202163157970 P
(43) Date of publication of application: 14.05.2025
(62) Document number(s) of the earlier application(s) in accordance with Art. 76 EPC: 22764119.8 / 4 301 285
(73) Proprietor: Shoulder Innovations, Inc., Grand Rapids, MI 49507 (US)
(72) Inventor: KIRITSIS, Paul, Midlothian, VA 23113 (US)
(74) Representative: Gill Jennings & Every LLP

(56) References cited:
- EP-B1- 0 965 312
- US-A1- 2007 173 945
- US-A1- 2012 041 485
- US-A1- 2015 190 237

## Description

### Cross-Reference to Related Applications

This application claims benefit of priority to U.S. Provisional Application No. 63/156,437, entitled "Implant Stem with Suture Retention Structure," filed March 4, 2021 and U.S. Provisional Application No. 63/157,970, entitled "Implant Stem with Suture Retention Structure," filed March 8, 2021.

### Background

The embodiments described herein generally relate to prosthetic implants with suture retention structures that can be used to secure tissues, bone, or muscles to the prosthetic implant. More specifically, the embodiments described herein relate to humeral stem implants with suture retention structures and methods for securing bone fragments from fracture or at least a portion of the subscapularis to the humeral stem implant and/or to the humerus.

Total shoulder replacement, also known as total shoulder arthroplasty (TSA), is a procedure where a damaged or diseased shoulder ball and socket joint are replaced with a prosthesis. In TSA procedures, the humeral articular surface is replaced with a stemmed humeral implant with a prosthetic ball head component or a socket typically used for a reverse total shoulder. In order to attach the subscapularis or fracture fragments back onto the humerus repaired with the stemmed humeral implant, sutures are passed through suture holes in the stemmed humeral implant and through holes drilled laterally into the humerus or around the bone fragments. However, the threading of sutures through suture holes of the stemmed humeral implant can be tedious, cumbersome and time consuming. Additionally, given the complexity of the anatomy and biomechanics of the shoulder joint, when two or more separate sutures are used to secure the subscapularis or fracture fragments to the stemmed humeral implant, the first suture tightened down will have a tendency to shift or require further tensioning after the second suture has been tightened down. This results in a surgeon having to repeat the tensioning process multiple times on at least one suture to reach an optimal level of tension across all the sutures used in the procedure.

EP 0 965 312 B1 relates to a metal endoprothesis which can be planted without cement. The (hip) implant has many grooves on the top surface and the rear section has grooves with a constant cross section. The grooves are transverse or perpendicular to the shaft longitudinal direction in the medial and proximal areas. Each groove is at a constant distance from each other.

Thus a need exists for an improved total shoulder arthroplasty implant and technique for tensioning and securing the subscapularis and/or fracture fragments to the humerus. Furthermore, a need exists to develop a new technique that can be performed with proficiency by a full spectrum of orthopedic surgeons who presently struggle with existing tensioning techniques.

### Summary

According to the present invention there is provided an implantable prosthesis according to claim 1. One or more preferred embodiments of the present invention are also defined in the appended claims. Implants with suture retention structures and methods for securing tissues or muscles to the implants are described herein. In some embodiments, an implantable prosthesis includes a stem and a stem head. The stem head may exist as a female socket to accept a male morse taper or may include the male portion of the morse taper which accepts a female socket. The stem extends along a longitudinal axis from a proximal end portion to a distal end portion of the stem. The stem head is coupled to the proximal end portion of the stem. The stem head is configured to be coupled to a joint component. At least one of the stem or the stem head includes a side surface and a side surface cleat. The side surface cleat defines a first side surface groove and a second side surface groove. The side surface cleat extends between the first side surface and the second side surface.

In some embodiments, an implantable prosthesis includes a stem and a stem head. The stem extends along a longitudinal axis from a proximal end portion to a distal end portion of the stem. The stem head is coupled to the proximal end portion of the stem. The stem head is configured to be coupled to a joint component. The stem head includes a joint mounting portion. The joint mounting portion includes a mounting surface extending along a mounting plane. The mounting plane and the longitudinal axis of the stem defines an acute angle therebetween. The mounting surface defines a first mounting surface groove and a second mounting surface groove. The mounting surface includes a mounting surface cleat extending between the first mounting surface groove and the second mounting surface groove.

In some embodiments, an implantable prosthesis includes a stem and a stem head. The stem extends along a longitudinal axis from a proximal end portion to a distal end portion of the stem. The stem head is coupled to the proximal end portion of the stem. The stem head is configured to be coupled to a joint component. The stem head includes a first side surface, a second side surface, and a passageway sidewall. The stem head defines a passageway that extends from the first side surface to the second side surface. At least a portion of the passageway intersects the longitudinal axis of the stem. The passageway is defined, at least in part, by the passageway sidewall. The passageway sidewall includes a passageway cleat and further defines a first passageway groove and a second passageway groove. The passageway cleat extends between the first passageway groove and the second passageway groove.

In some embodiments, not part of the present invention, a method of securing a portion of tissue or bone fragments to a bone or a humeral implant includes partially inserting an implantable prosthesis into a bore that has been produced within the bone. The implantable prosthesis includes a stem and a stem head coupled to the stem. The stem head includes a side surface and a joint mounting portion that is separate from the side surface. The joint mounting portion includes a joint mounting surface cleat. The method further includes inserting a first mounting loop through the portion of tissue, the first mounting loop being coupled to a cinching fixation system. The method includes securing the first mounting loop to the joint mounting surface cleat.

In some embodiments, not part of the present invention, a method of securing a portion of tissue or bone fragments to a bone or a prosthesis implant includes aligning the prosthesis implant with the bone. The prosthesis implant includes a joint mounting portion, a first cleat and a second cleat. The joint mounting portion including at least one of the first cleat or the second cleat. A first mounting loop is inserted through the portion of the tissue. The first mounting loop and a second mounting loop are each coupled to a cinching fixation system. The first mounting loop is secured to the first cleat. The second mounting loop is secured to the second cleat. The prosthesis implant is coupled to the bone. The method then includes pulling at least one tensioning end of the cinching fixation system to apply tension around the portion of tissue against the bone.

In some embodiments, the other of the first cleat or the second cleat can be included on a side surface or other portion of the prosthesis implant.

In some embodiments, the implantable prosthesis is a stemless implant. The aligning the prosthesis implant includes placing a contact surface of the stemless implant into contact with a resected surface of the bone. The coupling the prosthesis implant to the bone includes fastening the contact surface of the stemless implant to the resected surface of the bone.

In some embodiments, an implantable prosthesis includes a first surface and a second surface opposite the first surface. The first surface is configured to be coupled to a surface of a bone (e.g., a resected surface of a humerus). The second surface is configured to be coupled to a joint component. The second surface defines a first groove and a second groove, and includes a cleat extending between the first groove and the second groove.

In some embodiments, a kit includes an implantable prosthesis and a cinching fixation system. The implantable prosthesis includes a first surface and a second surface opposite the first surface. The first surface is configured to be coupled to a surface of a bone (e.g., a resected surface of a humerus). The second surface is configured to be coupled to a joint component. The second surface defines a first groove and a second groove, and includes a cleat extending between the first groove and the second groove. The cinching fixation system includes a first mounting loop and a second mounting loop each being coupled to an adjustable suture component (also referred to as a tensioning member). The first mounting loop is configured to be secured to the cleat and the second mounting loop including a bone anchor configured to couple the second mounting loop to the bone.

### Brief Description of the Drawings

FIG. 1 is a front view of a joint of a human musculoskeletal system.
FIG. 2 is a front view of an implantable prosthesis according to an embodiment.
FIG. 3 is an enlarged first side view of the implantable prosthesis of FIG 2.
FIG. 4 is an enlarged second side view of the implantable prosthesis of FIG 2.
FIG. 5 is an enlarged perspective view of the implantable prosthesis of FIG 2.
FIGS. 6A and 6B are cross-sectional views of grooves and cleats of the implantable prosthesis of FIG. 2, taken along line A-A shown in FIG. 3.
FIG. 7A is a cross-sectional view of grooves and a cleat of an implantable prosthesis according to an embodiment that has an asymmetric undercut configuration.
FIG. 7B is a cross-sectional view of grooves and a cleat of an implantable prosthesis according to an embodiment that has an asymmetric undercut configuration.
FIG. 7C is a cross-sectional view of grooves and a cleat of an implantable prosthesis according to an embodiment that has an asymmetric double undercut configuration.
FIGS. 8A-8C show cross-sectional views of grooves and cleats the implantable prosthesis of FIG. 2 during insertion of a suture into one of the grooves.
FIG. 9A is a cross-sectional view of grooves and a cleat of an implantable prosthesis according to an embodiment that has an angled tapered profile.
FIG. 9B is a cross-sectional view of a portion of an implantable prosthesis according to an embodiment that has at least one cleat and at least one through hole for securing a suture.
FIG. 10 is a cross-sectional view of a cleat of the implantable prosthesis of FIG. 2, taken along line B-B shown in FIG. 2.
FIG. 10A is a cross-sectional view of a cleat of an implantable prosthesis according to an embodiment.
FIG. 10B is a cross-sectional view of a cleat of an implantable prosthesis according to an embodiment.
FIG. 11 is a front view of an implantable prosthesis according to an embodiment.
FIG. 12 is a perspective view of an implant and tissue prior to being secured to a bone.
FIG. 13 is a perspective view of an implant and tissue being secured to a bone.
FIG. 13A is an enlarged perspective view of the bone in FIG. 13 after a portion of a mounting loop is positioned back into a bone tunnel.
FIG. 14 is a perspective view of an implant and tissue being secured to a bone.
FIG. 15 is a perspective view of an implant and tissue being secured to a bone.
FIG. 16 is a perspective view of tissue prior to being secured to a bone via an implant according to an embodiment.
FIG. 17 is a perspective view of tissue being secured to a bone.
FIG. 18 is a flow chart showing a method of securing tissue to an implant in a patient.
FIG. 19A is a second side view of an implantable prosthesis according to an embodiment.
FIG. 19B is an enlarged cross sectional view of the implantable prosthesis, taken along line X-X shown in FIG. 19A.
FIG. 20A is a second side view of an implantable prosthesis according to an embodiment.
FIG. 20B is an enlarged cross sectional view of the implantable prosthesis, taken along line X-X shown in FIG. 20A.

### Detailed Description

As used in this specification, specific words chosen to describe one or more embodiments and optional elements or features are not intended to limit the invention. For example, spatially relative terms-such as "beneath", "below", "lower", "above", "upper", "proximal", "distal", and the like-may be used to describe the relationship of one element or feature to another element or feature as illustrated in the figures. These spatially relative terms are intended to encompass different positions (i.e., translational placements) and orientations (i.e., rotational placements) of a device in use or operation in addition to the position and orientation shown in the figures. For example, if a device in the figures is turned over, elements described as "below" or "beneath" other elements or features would then be "above" or "over" the other elements or features. Thus, the term "below" can encompass both positions and orientations of above and below. A device may be otherwise oriented (e.g., rotated 90 degrees or at other orientations) and the spatially relative descriptors used herein interpreted accordingly. Likewise, descriptions of movement along (translation) and around (rotation) various axes includes various spatial device positions and orientations.

Similarly, geometric terms, such as "parallel", "perpendicular", "round", or "square", are not intended to require absolute mathematical precision, unless the context indicates otherwise. Instead, such geometric terms allow for variations due to manufacturing or equivalent functions. For example, if an element is described as "round" or "generally round," a component that is not precisely circular (*e.g*., one that is slightly oblong or is a many-sided polygon) is still encompassed by this description.

In addition, the singular forms "a", "an", and "the" are intended to include the plural forms as well, unless the context indicates otherwise. The terms "comprises", "includes", "has", and the like specify the presence of stated features, steps, operations, elements, components, etc. but do not preclude the presence or addition of one or more other features, steps, operations, elements, components, or groups.

As used herein, the term "about" when used in connection with a referenced numeric indication means the referenced numeric indication plus or minus up to 10% of that referenced numeric indication. For example, the language "about 50" covers the range of 45 to 55. Similarly, the language "about 5" covers the range of 4.5 to 5.5.

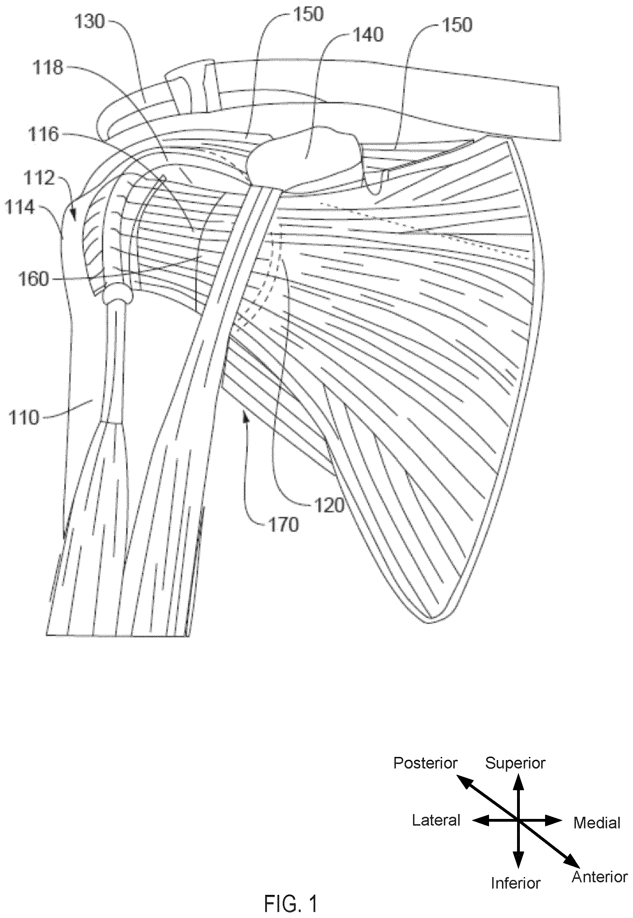

FIG. 1 is a front view of a joint of a human musculoskeletal system 100 including a humerus 110, a glenoid 120, an acromion 130, and a coracoid process 140. The joint further includes rotator cuff muscles and tendons 150, a subscapularis muscle 160, and an infraspinatus muscle 170 (located on a posterior side of the body). The humerus 110 includes a proximal humerus portion 112, a greater tuberosity 114, a lesser tuberosity 116 (which is on the anterior face of the humerus), and a humeral head 118. The glenoid 120 includes a cupped surface for interfacing with a curvature of the humeral head 118.

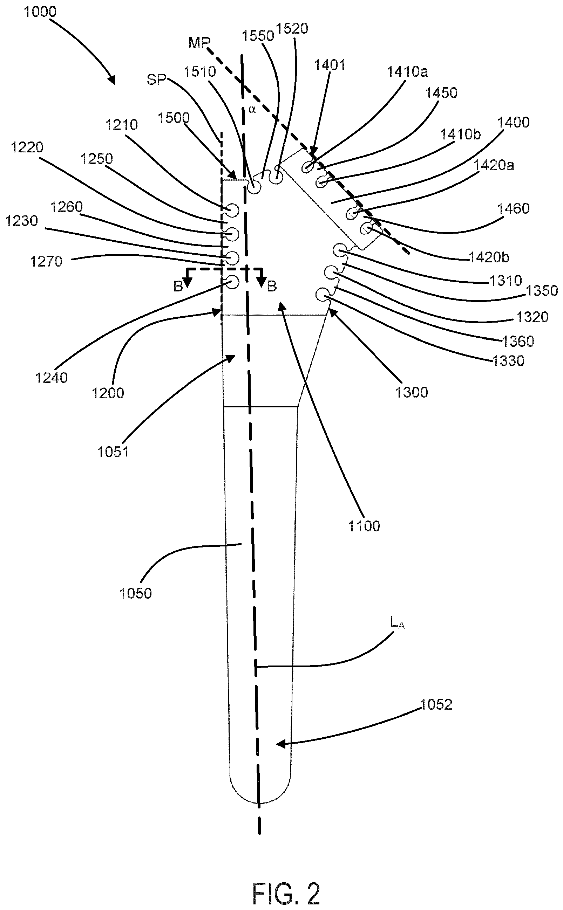

FIGS. 2-6 show an implantable prosthesis 1000 for repairing a bone, such as a humerus 110 (upper arm bone), a femur (thigh bone), or any other suitable bone. The implantable prosthesis 1000 includes a stem 1050 extending along a longitudinal axis L_{A} from a proximal end portion 1051 to a distal end portion 1052. The stem 1050 tapers from the proximal end portion 1051 to the distal end portion 1052 such that a cross-sectional area of the distal end portion 1052 is smaller than a cross-section area of the proximal end portion 1051. In some embodiments, the distal end portion 1052 has a circular, oval, rectangular, or rounded shape. As described herein, the distal end portion 1052 of the stem is configured to be inserted into a bore that has been reamed into or otherwise produced within a bone (e.g., the humerus).

The implantable prosthesis 1000 includes a stem head 1100 coupled to the proximal end portion 1051 of the stem 1050. The stem head 1100 is configured to be coupled to a joint component, such as a humeral head prosthesis, also referred to herein as a joint component. The stem head 1100 includes a first side surface 1200 (which may be referred to as a lateral side surface), a second side surface 1300 (which may be referred to as a medial side surface), and a joint mounting portion 1400. The stem head further includes a third side surface 1500 (which may be referred to as a superior side surface) extending between the first side surface 1200 and the joint mounting portion 1400. In some embodiments, the stem head 1100 (and any of the stem heads described herein) can constructed separately from and coupled to the stem 1050. In other embodiments, the stem head 1100 (and any of the stem heads described herein) and the stem 1050 can be monolithically constructed.

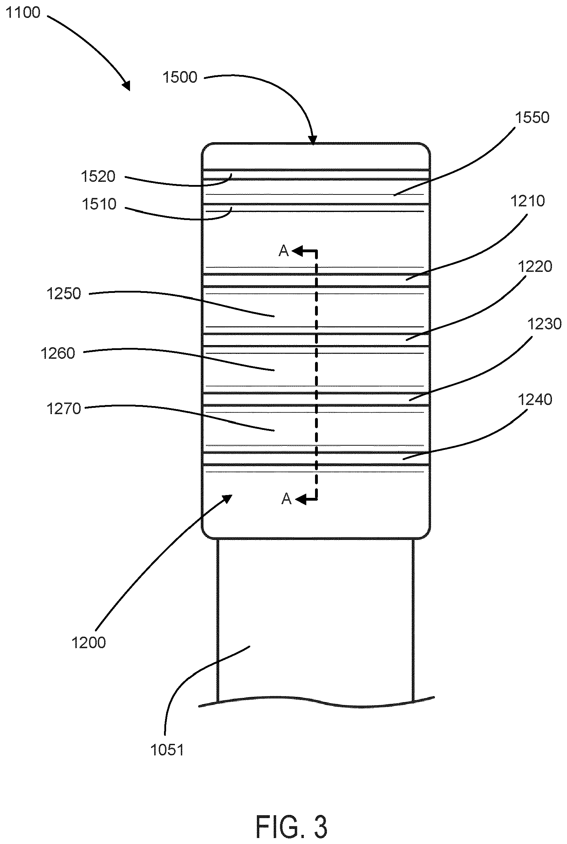

As shown in FIGS. 2 and 3, the first side surface 1200 defines a first side surface groove 1210, a second side surface groove 1220, a third side surface groove 1230, and a fourth side surface groove 1240. The stem head 1100 includes a first cleat 1250 of the first side surface 1200, a second cleat 1260 of the first side surface 1200, and a third cleat 1270 of the first side surface 1200. The first cleat 1250 extends between the first side surface groove 1210 and the second side surface groove 1220. The second cleat 1260 extends between the second side surface groove 1220 and the third side surface groove 1230. The third cleat 1270 extends between the third side surface groove 1230 and the fourth side surface groove 1240. Thus, each of the first cleat 1250, the second cleat 1260, and the third cleat 1270 are bounded, at least in part, by the adjacent grooves and therefore provide a protrusion or fixation structure to which a suture can be attached. For example, as described herein, in some embodiments, a suture loop can be placed (or wrapped) about at least one of the first cleat 1250, the second cleat 1260, and/or the third cleat 1270 as a part of a medical procedure. Although the first side surface 1200 is shown as defining four grooves and including three cleats, in other embodiments, a stem head can define any suitable number of grooves (and cleats extending therebetween). For example in some embodiments, the first side surface 1200 can define two grooves and include only one cleat or three grooves with two cleats. In yet other embodiments, the first side surface 1200 need not define any grooves or include any cleats, but rather the sutures, tapes, and/or mounting loops can be secured on cleats in other portions of the prosthesis 1000. For example, in some embodiments, a portion of the stem 1050 can include a set of grooves and one or more cleats. In other embodiments, the first side surface 1200 can be devoid of any grooves and cleats, and such fixation structures (i.e., cleats or protrusions) can instead be on the second side surface 1300 and/or the third side surface 1500.

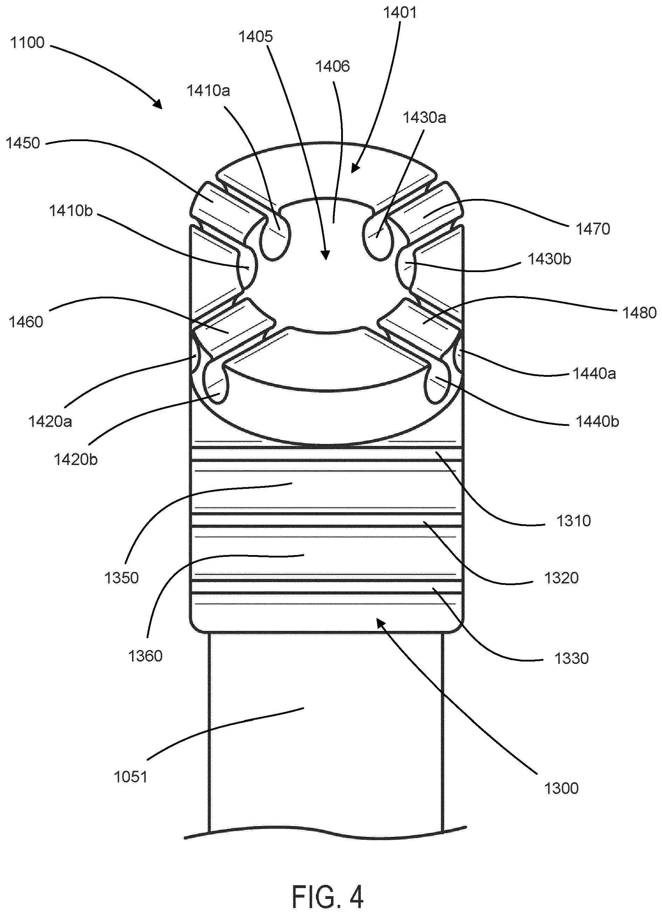

As shown in FIGS. 2 and 4, the second side surface 1300 defines a fifth side surface groove 1310, a sixth side surface groove 1320, and a seventh side surface groove 1330. The stem head 1100 includes a first cleat 1350 of the second side surface 1300, a second cleat 1360 of the second side surface 1300. The first cleat 1350 extends between the fifth side surface groove 1310 and the sixth side surface groove 1320. The second cleat 1360 extends between the sixth side surface groove 1320 and the seventh side surface groove 1330. Thus, each of the first cleat 1350 and the second cleat 1360 are bounded, at least in part, by the adjacent grooves and therefore provide a protrusion or fixation structure to which a suture can be attached. For example, as described herein, in some embodiments, a suture loop can be placed (or wrapped) about at least one of the first cleat 1350 of the second side surface 1300 and/or the second cleat 1360 of the second side surface 1300 as a part of a medical procedure. Although the second side surface 1300 is shown as defining three grooves and including two cleats, in other embodiments, a stem head can define any suitable number of grooves (and cleats extending therebetween). For example in some embodiments, the second side surface 1300 can define two grooves and include one cleat or four grooves with three cleats. In yet other embodiments, the second side surface 1300 need not define any grooves or include any cleats, but rather the sutures, tapes, and/or mounting loops can be secured on cleats in other portions of the prosthesis 1000 (e.g., via the cleats of the first side surface 1200).

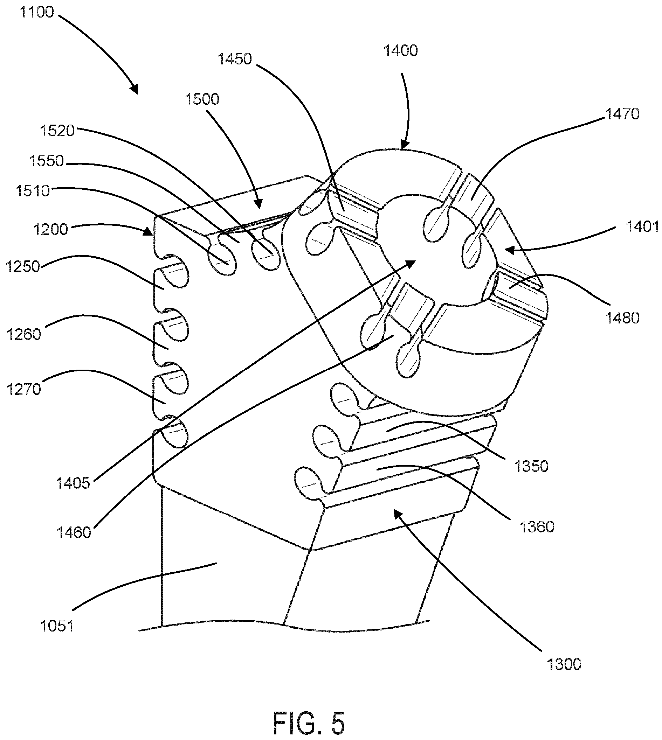

As shown in FIGS. 2 and 5, the joint mounting portion 1400 includes a mounting surface 1401 that extends along a mounting plane MP that is at an angle relative the longitudinal axis L_{A} of the stem 1050. The mounting plane MP and the longitudinal axis L_{A} define an acute angle α therebetween. The acute angle α is selected to provide the desired geometric and/or kinematic characteristics of the joint component (not shown) that is attached to the joint mounting portion 1400. In some embodiments, the acute angle α defined between mounting plane MP and the longitudinal axis L_{A} is between about 30 degrees to about 60 degrees. Although the mounting surface 1401 is shown and described as being planar, in other embodiments, the mounting surface can have any suitable shape. For example, in some embodiments, the mounting surface 1401 (or any of the mounting surfaces described herein) can have a cupped (or concave) shape, or alternatively a domed (or convex) shape. In such embodiments, the acute angle α is defined between a line that is tangent to a portion the mounting surface 1401 and the longitudinal axis L_{A} of the stem 1050.

The joint mounting portion 1400 includes a joint component retention mechanism 1405. As shown in FIG. 5, the joint component retention mechanism 1405 includes a recess 1406 for receiving a protrusion of a joint component (not shown). In some embodiments, the joint component retention mechanism 1405 includes a female portion of a fastener. For example, the female portion can include female screw threads for receiving male screw threads of a joint component. In some embodiments, the retention mechanism 1405 includes a male portion of a fastener. For example, the male portion can include male screw threads for supporting female screw threads of a joint component.

The mounting surface 1401 defines a first mounting surface groove 1410a, a second mounting surface groove 1410b, a third mounting surface groove 1420a, a fourth mounting surface groove 1420b, a fifth mounting surface groove 1430a, a sixth mounting surface groove 1430b, a seventh mounting surface groove 1440a, and an eighth mounting surface groove 1440b. The stem head 1100 and/or the mounting surface 1401 includes a first mounting surface cleat 1450 extending between the first and second mounting surface grooves 1410a, 1410b. The stem head 1100 and/or the stem head 1100 and/or the mounting surface 1401 includes a second mounting surface cleat 1460 extending between the third and fourth mounting surface grooves 1420a, 1420b. The stem head 1100 and/or the mounting surface 1401 includes a third mounting surface cleat 1470 extending between the fifth and sixth mounting surface grooves 1430a, 1430b. The stem head 1100 and/or the mounting surface 1401 includes a fourth mounting surface cleat 1480 extending between the seventh and eighth mounting surface grooves 1440a, 1440b. Thus, each of the first mounting surface cleat 1450, the second mounting surface cleat 1460, the third mounting surface cleat 1470, and the fourth mounting surface cleat 1480 are bounded, at least in part, by the adjacent grooves and therefore provide a protrusion or fixation structure to which a suture can be attached. For example, as described herein, in some embodiments, a suture loop can be placed (or wrapped) about at least one of the mounting surface cleats as a part of a medical procedure. Although the mounting surface 1401 is shown as defining four pairs of grooves and including four cleats, in other embodiments, the mounting surface 1401 can define any suitable number of grooves (and cleats extending therebetween). For example in some embodiments, the mounting surface 1401 can define two pairs of grooves and include two cleats. In yet other embodiments, the mounting surface 1401 need not define any grooves or include any cleats, but rather the sutures, tapes, and/or mounting loops can be secured on cleats in other portions of the prosthesis 1000 (e.g., via the cleats of the first side surface 1200 or the second side surface 1300).

Referring back to FIG. 2, the third side surface 1500 defines an eighth side surface groove 1510 and a ninth side surface groove 1520. The stem head 1100 and/or the third side surface 1500 includes a cleat 1550. The cleat 1550 extends between the eighth side surface groove 1510 and the ninth side surface groove 1520. Although the third side surface 1500 is shown as defining two grooves and including one cleat, in other embodiments, the third side surface 1500 can define any suitable number of grooves (and cleats extending therebetween). In other embodiments, the third side surface 1500 need not define any grooves or include any cleats, but rather the sutures, tapes, and/or mounting loops can be secured on cleats in other portions of the prosthesis 1000 (e.g., via the cleats of the first side surface 1200).

As described above, a prosthesis can include any number of cleats. While the first side surface 1200 is depicted with three cleats 1250, 1260, 1270 and four corresponding side surface grooves 1210, 1220, 1230, 1240, in some embodiments, the first side surface 1200 includes between one and five (or more) cleats. Similarly, while the second side surface 1300 is depicted with two cleats 1350, 1360 and three corresponding side surface grooves 1310, 1320, 1330, in some embodiments, the second side surface 1300 includes between one and four (or more) cleats. Additionally, while the mounting surface 1401 is depicted with a total of four cleats 1450, 1460, 1470, 1480, in some embodiments, the mounting surface 1401 includes between one and eight (or more) cleats. In some embodiments, the stem head 1100 includes cleats only on the first side surface 1200 and on the second side surface 1300. In some embodiments, the stem head 1100 includes cleats only on the first side surface 1200 and on the mounting surface 1401. In some embodiments, the stem head 1100 includes cleats only on the mounting surface 1401 and the third side surface 1500. In some embodiments, the stem head 1100 includes cleats only on the first side surface 1200, on the second side surface 1300, and on the mounting surface 1401.

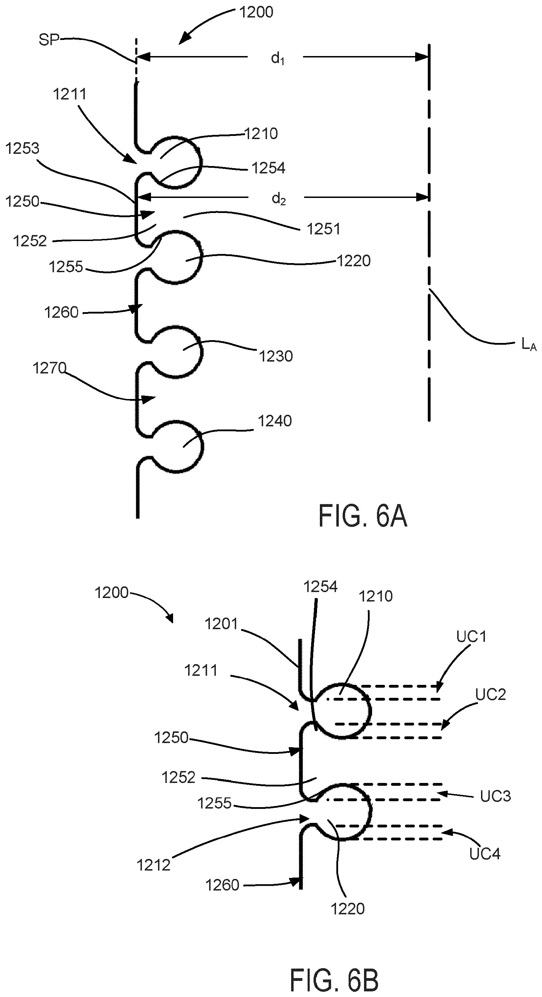

The cleats of the implantable prosthesis 1000 and any of the prosthesis implants described herein can have any suitable shape, size, and/or structure to provide the desired fixation properties for securing a suture or other flexible member to the prosthesis implant during a procedure. For example any of the cleats described here can formed between adjacent (or surrounding) grooves or recesses to form a protrusion to which a suture can be attached. For example, FIGS. 6A and 6B show an enlarged cross-sectional view of grooves and cleats of the first side surface 1200 taken at line A-A in FIG. 3. The first side surface 1200 defines a side surface plane SP (see also FIG. 2). The side surface plane SP is spaced a first distance d₁ from the longitudinal axis L_{A} of the stem 1050 (see FIG. 6A). The first cleat 1250 includes a base portion 1251 and a retention portion 1252 that is coupled to the base portion 1251. In some embodiments, an outer surface 1253 of the retention portion is spaced a second distance d₂ from the longitudinal axis L_{A} of the stem 1050, where the second distance is less than or equal to the first distance. In other words, an outer surface 1253 of the retention portion 1252 is flush with the side surface plane SP, or is recessed inward towards the stem head 1100 relative to the side surface plane SP. Similarly stated, the outer surface 1253 (and therefore the first cleat 1250) do not protrude from or extend beyond the side surface plane SP. In this manner, the first cleat 1250 (and/or any of the other cleats described herein) will not protrude outside of the prosthesis envelope or otherwise disrupt the process of inserting the prosthesis 1000 into the bone. By providing the cleats within the overall prosthesis envelope, the embodiments described herein do not complicate the implant procedure. Although the side surface plane SP is illustrated as being parallel with the longitudinal axis L_{A} of the stem 1050, in some embodiments, the side surface plane as shown in FIG. 6 can be offset about 5 degrees to about 30 degrees relative to the longitudinal axis L_{A} of the stem 1050. Similarly stated, although FIG. 6A shows the distances d₁ and d₂ as being perpendicular to the longitudinal axis L_{A} of the stem 1050 and the side surface plane SP, in other embodiments, the distances d₁ and d₂ can be at any suitable angle with respect to the longitudinal axis L_{A} and or the side surface plane SP.

The retention portion 1252 includes a first inner surface 1254 and a second inner surface 1255. The first inner surface 1254 and the second inner surface 1255 define an undercut portion of the first side surface groove 1210 and the second side surface groove 1220, respectively. Although not fully labeled in FIG. 6A, the second cleat 1260 and the third cleat 1270 similarly include respective base portions and retention portions. Additionally, any of the cleats described herein can include a base portion and a retention portion similar to the one described with reference to the first cleat 1250. In some embodiments, as generally shown in FIG. 6B, a first portion 1201 of the side surface 1200 and the retention portion 1252 of the cleat 1250 define a first opening 1211 into the first side surface groove 1210. A retention portion of the cleat 1260 and the retention portion 1252 of the cleat 1250 define a second opening 1212 into the second side surface groove 1220. As shown in FIG. 6B, an inner portion opposite the first portion of the side surface 1200 defines a first undercut portion UC1 of the first side surface groove 1210. The first inner surface 1254 of the single cleat 1250 defines a second undercut portion UC2 of the first side surface groove 1210. The second inner surface 1255 of the single cleat 1250 defines a third undercut portion UC3 of the second side surface groove 1220. An inner portion opposite the second portion of the side surface 1200 defines a fourth undercut portion UC4 of the second side surface groove 1220. Although the first undercut portion UC1, the second undercut portion UC2, the third undercut portion UC3, and the fourth undercut portion UC4 are depicted as having a similar size (e.g., width) and shape, in some embodiments, a size (e.g., width) of the first undercut portion UC1 and fourth undercut portion UC4 is greater than a width of the second undercut portion UC2 and the third undercut portion UC3. In some embodiments, width of the first undercut portion UC1 and fourth undercut portion UC4 is less than a width of the second undercut portion UC2 and the third undercut portion UC3. In this manner the undercut that forms a portion of the cleats described herein can be sized and shape to provide the desired fixation characteristics. For example, after the suture, tape, loop, or tensioning member has been passed into the first side surface groove 1210, the first inner surface 1254 (including the size and shape of the first undercut portion UC1) and/or other similar structure that defines the first side surface groove 1210 prevents the suture, tape, loop, or tensioning member from being passed back out.

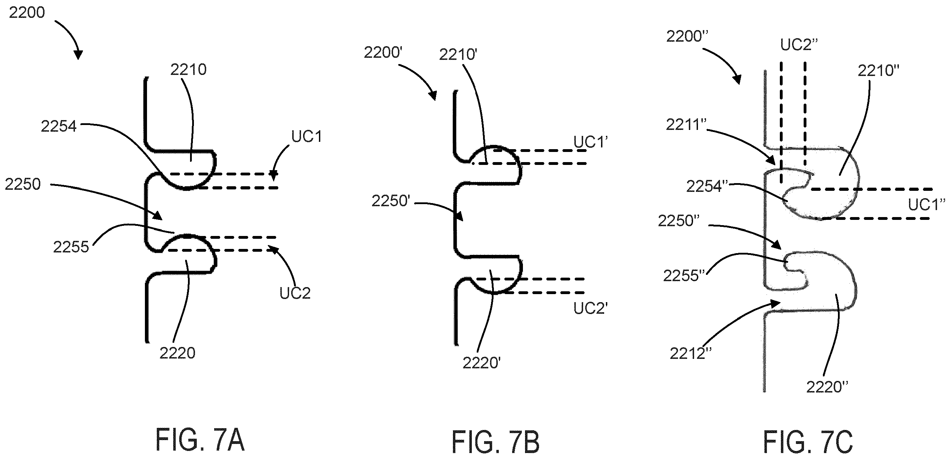

In some embodiments, the opening into a groove can be asymmetrical and/or can produce an asymmetric undercut. As one example, FIG. 7A shows an embodiment having a side surface 2200 that includes a first side surface groove 2210, a second side surface groove 2220, and a single cleat 2250 extending between the first and second side surface groove 2210, 2220. A first inner surface 2254 of the single cleat 2250 defines a first undercut portion UC1 of the first side surface groove 2210. A second inner surface 2255 of the single cleat 2250 defines a second undercut portion UC2 of the second side surface groove 2220. The portions of the side surface 2200 abutting the first side surface groove 2210 or the second side surface groove 2220 do not include any undercut portions.

In some embodiments, as generally shown in FIG. 7B, a side surface 2200' includes a first side surface groove 2210', a second side surface groove 2220', and a single cleat 2250' extending between the first and second side surface grooves 2210', 2220'. An inner portion opposite the first portion of the side surface 2200' defines a first undercut portion UC1' of the first side surface groove 2210'. An inner portion opposite the second portion of the side surface 2200' defines a second undercut portion UC2' of the second side surface groove 2210'. In this embodiment, the single cleat 2250' does not include any undercut in the depicted cross-section. Instead, the undercut portions are formed in adjacent portions of the side surface 2200'.

In some embodiments, the opening into and of the grooves can be offset from the main portion of the groove. Additionally, although FIGS. 7A and 7B show one or more surfaces that that define a single undercut, in other embodiments, the surface defining the opening and/or a portion of the groove can include multiple undercuts. For example, FIG. 7C shows a portion of an implant having a side surface 2200" that defines a first side surface groove 2210" and a second side surface groove 2220", with a first opening 2211" into the first side surface groove 2210" and a second opening 2212" into the second side surface groove 2220". Thus, the implant includes a cleat 2250" extending between the first and second side surface grooves. As shown, the first opening 2211" is offset from (or asymmetrical with) the main portion of the first side surface groove 2210" and the second opening 2212" is offset from (or asymmetrical with) the main portion of the second side surface groove 2220". Additionally, a first inner surface 2254" of the cleat 2250 defines a first undercut portion UC1" and a second undercut portion UC2". A second inner surface 2255" of the cleat 2250" similarly defines two undercut portions. The offset arrangement of the openings and the two undercut configuration produce the cleat 2250" having portions that capture and retain the suture when wrapped about the cleat.

Referring again to FIGS. 6A and 6B, a portion of the first side surface 1200 and the retention portion 1252 of the first cleat 1250 define an opening 1211 into the first side surface groove 1210. Similarly, the first side surface 1200 defines an opening 1212 into the second side surface groove 1220 and similar openings into the third side surface groove 1230 and the fourth side surface groove 1240. As shown, the openings into the side surface grooves have a cross-sectional profile that is tapered, beveled, and/or chamfered. In some embodiments, the openings have a rounded profile that narrows upon entry into the side surface grooves. In this manner, the shape of the first side surface 1200 (and any of the other side surfaces described herein) can facilitate easily passing the suture, tape, loop, or tensioning member through the openings (e.g., the opening 1211 and 1212) and into the side surface grooves (e.g., the side surface groove 1210 and the side surface groove 1220). In some embodiments, one or more of the surfaces associated with the openings, grooves, and/or retention portions described herein may include surface treatments to enable the suture or tensioning members to slide into and/or to shift within the groove with less resistance. The surface treatments may also prevent damage to the suture or tensioning members while the suture or tensioning members are being attached around the cleats and placed into the respective grooves. For example, the one or more surfaces can be polished, machined, or coated to remove sharp edges and/or to refine rough or abrasive surfaces.

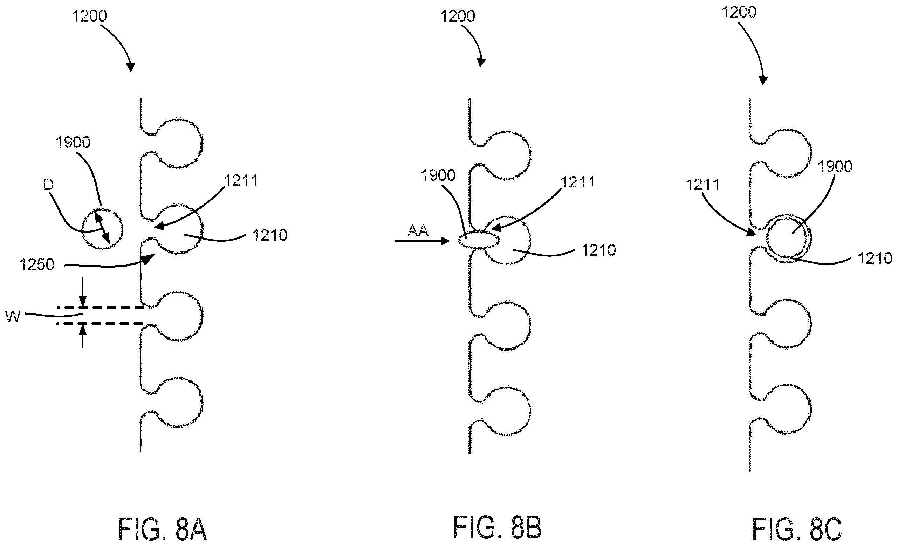

In some embodiments, the cross-sectional profile of the opening is configured to compress a portion of the suture, tape, loop, or tensioning member as it passes through the opening and into the first side surface groove. For example, FIGS. 8A-8C show a cross-sectional view of the side surface 1200 and a portion of a suture 1900 during various stages of being inserted through the opening 1211 and into the first side surface groove 1210. The suture 1900 can be a portion of any of the fixation systems described herein, such as, for example a mounting loop (e.g., the mounting loop 5010 described below). As shown in FIG. 8A, the width W (or size) of the smallest portion of the opening 1211 is smaller than the nominal diameter D of the suture 1900. Note that the width W is shown on an opening adjacent to the opening 1211 for clarity. Similarly stated, a size of the opening is smaller than a nominal size of the suture, tape, loop, or tensioning member. In use, the suture 1900 can be passed through the opening 1211, as shown by the arrow AA in FIG. 8B. The smaller size and the shape of the opening 1211 can compress and/or deform the suture 1900 to allow the suture to pass through the opening 1211 and into the first side surface groove 1210. As described below, the tapered or under-sized openings will retain the sutures or fastening members within the grooves, thereby preventing them from slipping out during any of the methods described herein.

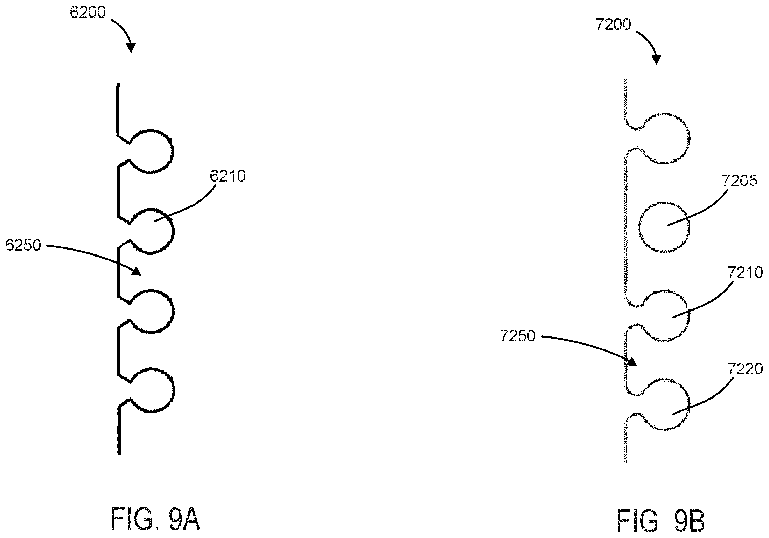

Although the opening 1211 into the side surface groove 1220 is shown and described as having a rounded profile, in other embodiments, any of the openings into any of the grooves of any of the implants described herein can have any suitable cross-sectional profile that is tapered, beveled, and/or chamfered. For example, FIG. 9A shows a portion of a side surface 6200 of an implant according to an embodiment. The side surface defines a groove 6210 and includes at least one cleat 6250. The side surface 6200 and structure thereof can be included in any of the implants described herein, such as, for example, the implants 1000 and 4000. As shown, the cross-sectional profile of the opening 6211 into the groove 6210 is V-shaped. As described above, the tapered, beveled, and/or chamfered cross-sectional profile permits a suture, tape, loop, or tensioning member to be passed sideways into the first side surface groove 6210. Once the suture, tape, loop, or tensioning member has been passed into the first side surface groove 6210, the shape of the opening (including the undercuts) prevents the suture, tape, loop, or tensioning member from being passed back out. As described above, any of the implants described herein can include double undercuts (see e.g., the implant shown in FIG. 7C) to improve the retention of the suture within the groove.

Although the side surface 1200 is shown as defining a set of grooves and including a set of cleats, in other embodiments, the side surface 1200 and any of the surfaces of any of the implants described herein can include both cleats and through holes. For example, FIG. 9B shows a cross-sectional view of a side surface 7200 of an implant according to an embodiment. The side surface 7200 and structure thereof can be included in any of the implants described herein, such as, for example, the implants 1000 and 4000. As shown, the side surface defines a first groove 7210 and a second groove 7220, and includes at least one cleat 7250. The side surface also defines a through hole 7205 through which a suture can be passed from either end of the implant. Similarly stated, in some embodiments, any of the implants described herein can include a groove or hole that is full enclosed by the end of the side surface. In this manner, the implant can facilitate methods where a suture is wrapped about a cleat (as described herein), as well as where a suture is passed through the hole (e.g., the hole 705).

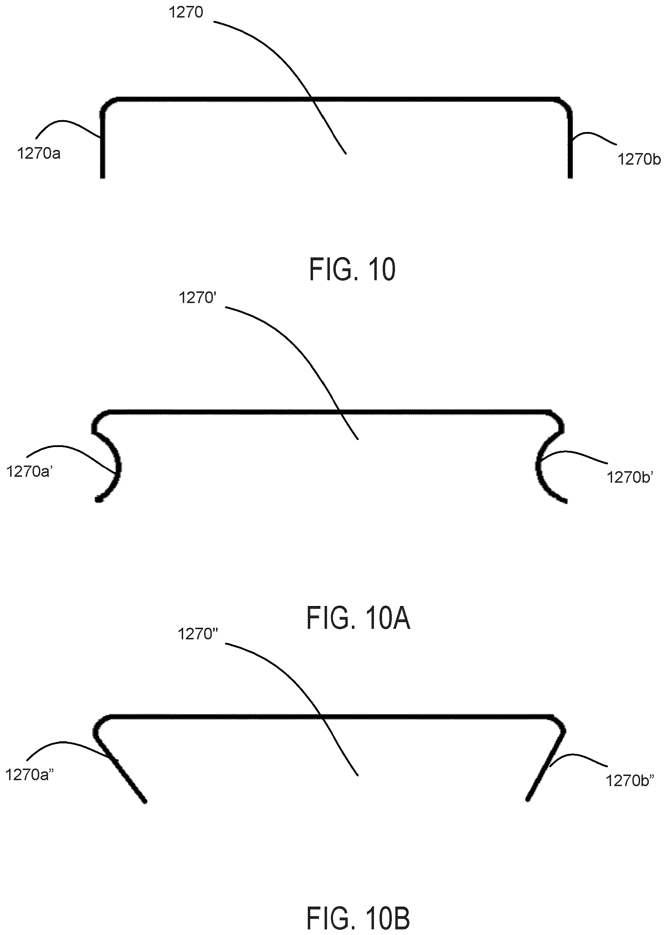

In some embodiments, the cleats can be shaped or tapered to facilitate guiding the suture or fastener into the grooves and around the cleats. For example, although FIGS. 6A and 6B illustrate the tapered profile within the cross-sectional plane shown by the line A-A in FIG. 3, in other embodiments, any of the other surfaces that define any of the cleats described herein can be rounded, tapered or chamfered to limit the likelihood of catching a suture on an edge. For example, FIG. 10 shows an enlarged cross-sectional view of the third cleat 1270 of the first side surface 1200 taken at line B-B in FIG. 2. The third cleat 1270, or any of the cleats described herein, can include linear end wall portions 1270a, 1270b. In some embodiments, as shown in FIG. 10A, a third cleat 1270', or any of the cleats described herein, can include concave or curvilinear end wall portions 1270a', 1270b'. In some embodiments, as shown in FIG. 10B, a third cleat 1270", or any of the cleats described herein, can include inwardly tapering end wall portions 1270a", 1270b".

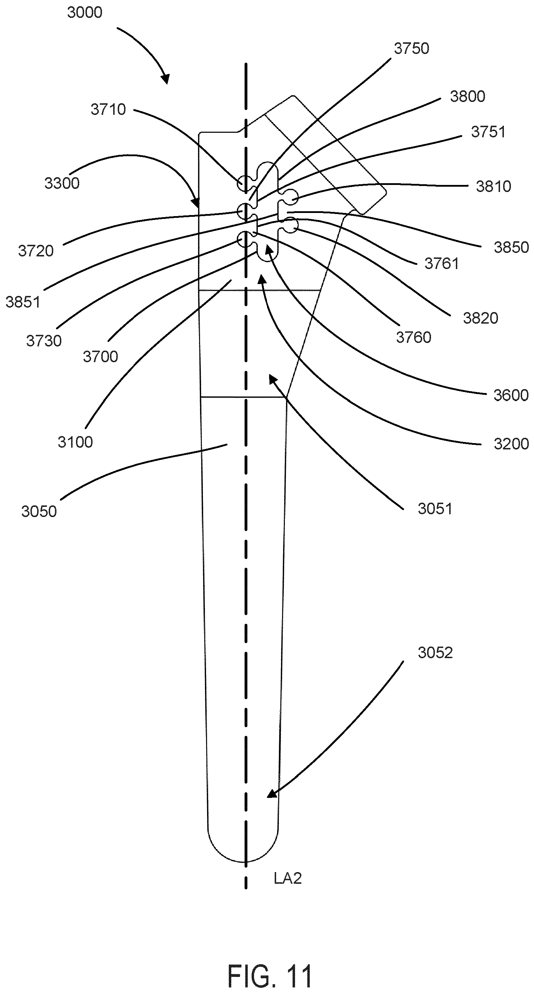

As described with reference to FIG. 9B, in some embodiments, and implant can include both cleats and through holes to facilitate fixation of a suture, tape, or tension member. In other embodiments an implant can include one or more openings therethrough that include internal cleats to which a suture, tape, or tension member can be affixed. Similarly stated, in some embodiments, any of the implants described herein can include one or more cleats that are fully surrounded by a portion of the implant (i.e., they are not exposed on a side edge of the implant). For example, FIG. 11 shows an implantable prosthesis 3000 including a stem 3050 extending along a longitudinal axis L_{A} from a proximal end portion 3051 to a distal end portion 3052. The stem 3050 tapers from the proximal end portion 3051 to the distal end portion 3052 such that a cross-sectional area of the distal end portion 3052 is smaller than a cross-section area of the proximal end portion 3051. In some embodiments, the distal end portion 3052 has a circular, oval, or rounded shape. As described herein, the distal end portion 1052 of the stem is configured to be inserted into a bore that has been produced within (e.g., reamed into) a bone (e.g., the humerus).

The implantable prosthesis 3000 includes a stem head 3100 coupled to the proximal end portion 3110 of the stem 3050. The stem head 3100 is configured to be coupled to a joint component, such as a humeral head prosthesis (also referred herein as a joint component). The stem head 3100 includes a first side surface 3200 (which may be referred to as an anterior side surface) and a second side surface 3300 (which may be referred to as a posterior side surface), and a joint mounting portion 3400. A passageway 3600 extends through the stem head 3100 from the first side surface 3200 to the second side surface 3300.

The passageway 3600 defines a first passageway sidewall 3700 and a second passageway sidewall 3800, the second passageway sidewall 3800 separate from the first passageway sidewall 3700. The first passageway sidewall 3700 defines a first passageway groove 3710, a second passageway groove 3720, and a third passageway groove 3730. The first passageway sidewall 3700 includes a first passageway cleat 3750 and a second passageway cleat 3760. The first passageway cleat 3750 extends between the first passageway groove 3710 and the second passageway groove 3720. The second passageway cleat 3760 extends between the second passageway groove 3720 and the third passageway groove 3730. In some embodiments, an outer surface 3751 of the first passageway cleat 3750 and/or an outer surface 3761 of the second passageway cleat 3760 is flush with the first passageway sidewall 3700.

The second passageway sidewall 3800 defines a fourth passageway groove 3810 and a fifth passageway groove 3820. The second passageway sidewall 3800 includes a third passageway cleat 3850. The third passageway cleat 3850 extends between the fourth passageway groove 3810 and the fifth passageway groove 3820. In some embodiments, an outer surface 3851 of the third passageway cleat 3850 is flush with the second passageway sidewall 3800. In some embodiments, the implantable prosthesis 3000 further includes a lateral side surface and a joint mounting portion, such as the first side surface 1200 and the joint mounting portion 1400 of the implantable prosthesis 1000. In some embodiments, the implantable prosthesis 3000 further includes a medial side surface, such as the second side surface 1300 of the implantable prosthesis 1000. The implantable prosthesis 3000 can include one or more cleats on one or more of the lateral side surface, the medial side surface, and or a mounting surface of the joint mounting portion.

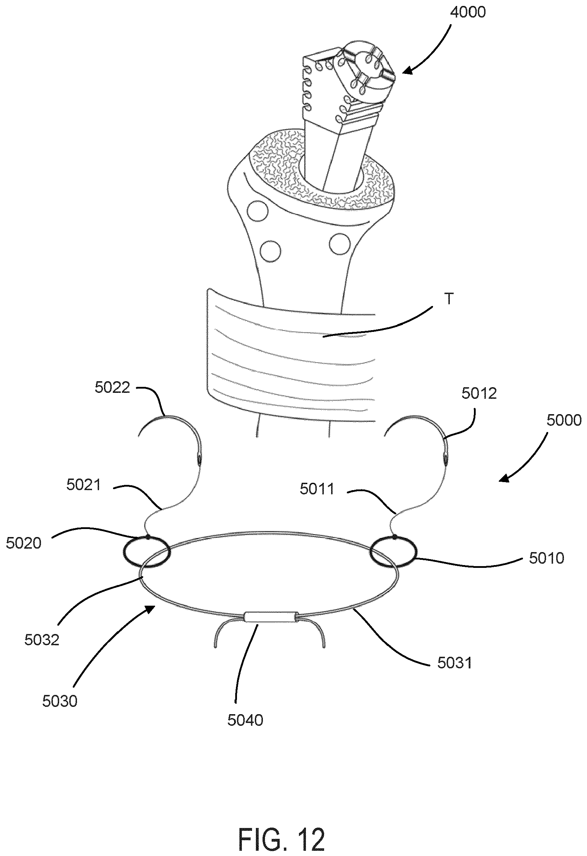

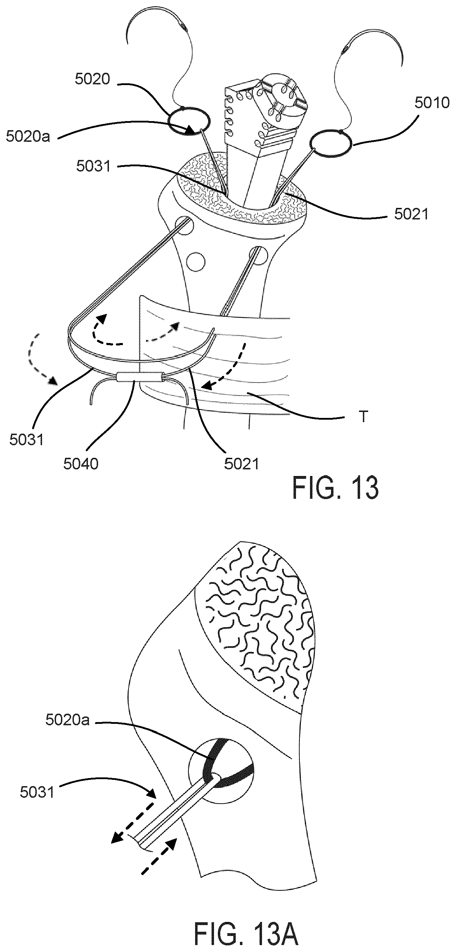

FIGS. 12 and 13 illustrate shoulder joint total shoulder arthroplasty (TSA) techniques using an implantable prosthesis 4000 and a cinching fixation system 5000 in accordance with aspects of the present disclosure. The implantable prosthesis 4000 can be any implantable prosthesis described herein, including the implantable prosthesis 1000 and the implantable prosthesis 3000. In some embodiments, an implant system (or kit) includes the implantable prosthesis 4000 and a cinching fixation system 5000. In some embodiments, the kit includes the implantable prosthesis 4000 and two to four cinching fixation systems 5000 to secure tissue or bone at multiple locations to a bone or to the implant 4000. In some embodiments, the cinching fixation system 5000 includes a first mounting loop 5010, a second mounting loop 5020, a tensioning member 5030, and a cinching member 5040 (e.g., a sheath, shroud, enclosure, or other structure to facilitate changing the length (or tightening) of the tensioning member 5030. The tensioning member 5030 and cinching member 5040 can be any suitable system to allow for an adjustable length, self-locking fastener. The use of such a tensioning member can reduce and/or eliminate the need to adjust the length and secure the tensioning member by manually pulling and tying knots. Examples of suitable tensioning members include Zimmer Biomet ZipLoop^{™} and Arthrex TightRope^{®}. As shown, the tensioning member includes a first tensioning end portion 5031 and a second tensioning end portion 5032. The first tensioning end portion 5031 is routed through the first mounting loop 5010 and routed through the cinching member 5040. The second tensioning end portion 5032 is routed through the second mounting loop 5020 and through the cinching member 5040. In use, the free ends of the tensioning member 5030 can be pulled through the cinching member 5040 to adjust the length and affix the tensioning member 5030.

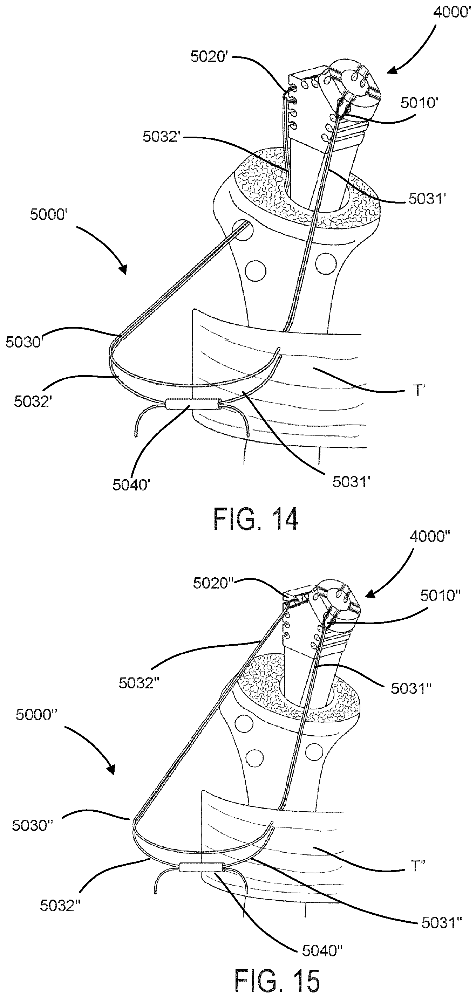

The following methods of performing TSA do not form part of the present invention. In some embodiments, a method of performing TSA includes resecting a portion of the humeral head of a humerus to form a diagonal surface. In some embodiments, the method includes reaming a proximal end portion of the humerus to form a bore. The method includes partially inserting an implantable prosthesis into the bore. As shown in FIG. 12, the first mounting loop 5010 is coupled to the tensioning member 5030 of the cinching fixation system 5000 and is inserted through a portion of tissue T or around bone using a suture 5011 and a needle 5012 attached to the first mounting loop 5010. The first mounting loop 5010 is then secured onto a first cleat of the implantable prosthesis. Specifically, the first mounting loop 5010 can be pushed through the openings and into the grooves to retain the loop about the first cleat, as described above (see also FIGS. 13 and 14). For example, the first cleat can be any cleat on the first side surface 1200, the second side surface 1300, the third side surface 1500, and/or the mounting surface 1401 of the implantable prosthesis 1000 described herein. Although FIG. 12 shows the stem of the implantable prosthesis 4000 being partially inserted into the bore, in other embodiments, the method need not include partially implanting the stem into the bore. Rather, in some embodiments, the method can include aligning the implantable prosthesis with a portion of the bone or the bore. For example, in some embodiments, a surgeon may maintain the implantable prosthesis fully out of the bore (but yet aligned or oriented with the bone or the bore) while completing the steps of pushing the mounting loops through the tissue and/or through any bone tunnels.

The method includes inserting the second mounting loop 5020, which is coupled to the tensioning member 5030, through a bone tunnel in a portion of the humerus and into the bore of the humerus. In some embodiments, a suture 5021 with a needle 5022 can be attached to the second mounting loop 5020 to facilitate passing the loop (and a portion of the tensioning member) through the bone tunnel. The second mounting loop 5020 is withdrawn out of the bore of the humerus (see, e.g., FIG. 13). The second mounting loop 5020 is then secured onto a second cleat of the implantable prosthesis. Specifically, the second mounting loop 5020 can be pushed through the openings and into the grooves to retain the loop about the second cleat. For example, the second cleat can be any cleat on the lateral surface 1200 or on the superior surface 1500 of the implantable prosthesis 1000. The second cleat can also be any other cleat shown and described herein. When the prosthesis is positioned as desired (e.g., placed fully within the bore), the method includes pulling at least one tensioning end of the cinching fixation system 5000 through the cinching member 5040 to apply tension around the portion of tissue against the humerus. By using a tensioning member that is securely coupled to the prosthesis, the tissue can be tensioned to the bone or prosthesis without the need for tying (and/or retying) knots. Thus, this procedure can facilitate greater efficiency in the TSA process or in fracture repair. In some applications, such as in reverse shoulder arthroplasty procedures with recent proximal humerus fractures, the method may include securing one or more fracture fragments to the humerus with the tensioning member in addition to securing the tissue to the humerus. In some embodiments, a separate tensioning member (different from the tensioning member used to secure the tissue) may be used to secure the one or more fracture fragments to the humerus.

In some embodiments, an end portion 5020a of either the second mounting loop 5020 (or any mounting loops described herein that are initially inserted through a bone tunnel) can be pulled back into the bone tunnel after being mounted to a cleat of the implantable prosthesis and the implantable prosthesis has been seated into the humerus. For example, as shown in FIG. 13A, as the implantable prosthesis is inserted and seated into the humerus, the end portion 5020a of the second mounting loop 5020 may be pulled back into the bone tunnel, such that the end portion 5020a of the second mounting loop 5020 is positioned within the bone tunnel or extends slightly out of the bone tunnel into the external environment. In this position, the end portion 5020a of the second mounting loop 5020 enables the second tensioning end portion 5032 to be more freely passed through the second mounting loop 5020 and then fed through to the cinching member 5040. In some embodiments, the tissue is the subscapularis. In some embodiments, the method includes securing tissue and/or fracture fragment to the humerus. In some embodiments, the bone tunnel is drilled through a portion of the humerus from a portion of the bicipital groove to the bore.

In some embodiments, as shown in FIG. 14, a first mounting loop 5010' is coupled to a tensioning member 5030' of a cinching fixation system 5000' and is inserted through a portion of tissue T' using a needle. The method then includes securing a first mounting loop 5010' directly onto a first cleat of the implantable prosthesis without passing the first mounting loop 5010' through a bone tunnel. For example, the first cleat can be any cleat on medial surface 1300 or on the mounting surface 1401 of the implantable prosthesis 1000 described herein. The method includes inserting a second mounting loop 5020' through a second bone tunnel formed through a portion of the humerus and into the bore of the humerus. The second mounting loop is coupled to the tensioning member 5030' of the cinching fixation system 5000'. The second mounting loop 5020' is withdrawn out of the bore of the humerus. The second mounting loop 5020' is then secured onto a second cleat of the implantable prosthesis. For example, the second cleat can be any cleat on the lateral surface 1200 or on the superior surface 1500 of the implantable prosthesis 1000. The pulling at least one tensioning end of the cinching fixation system 5000' through the cinching member 5040' to apply tension around the portion of tissue against the humerus. In some embodiments, the tissue is the subscapularis. In some embodiments, the bone tunnel is drilled through a portion of the humerus from a portion of the bicipital groove to the bore.

In some embodiments, none of the mounting loops and the tensioning end portions need not be passed through a bone tunnel. As shown in FIG. 15, a first mounting loop 5010" is coupled to a tensioning member 5030" of a cinching fixation system 5000" and is inserted through a portion of tissue T' using a needle. The first mounting loop 5010" is then secured onto a first cleat of the implantable prosthesis. For example, the first cleat can be any cleat on the mounting surface 1401 of the implantable prosthesis 1000 described herein. Optionally, in some embodiments, the method includes wrapping the tensioning member 5030" around a fracture fragment. The second mounting loop 5020" is then secured onto a second cleat of the implantable prosthesis. For example, the second cleat can be any remaining cleat on the mounting surface 1401 or a cleat on the superior side surface 1500. When the implantable prosthesis is positioned as desired (e.g., placed fully within the bore), the method includes pulling a first tensioning end portion 5031" and a second tensioning end portion 5032" of the tensioning member 5030" in a generally anterior direction to remove slack and to pull the first mounting loop 5010" and the second mounting loop 5020" to a side edge of the humerus. A joint component is then mounted to the implantable prosthesis such that the first mounting loop 5010" and the second mounting loop 5020" are sandwiched and secured between the joint component and the proximal end surface of the humerus.

In some embodiments, the method includes pulling at least one tensioning end of the cinching fixation system 5000" through the cinching member 5040" to apply tension around the portion of tissue against the humerus. In some embodiments, the tissue is the subscapularis. In some embodiments, the bone tunnel is drilled through a portion of the humerus from a portion of the bicipital groove to the bore.

As will be appreciated by one skilled in the art, the techniques generally shown and described with reference to FIGS. 13-15 can be repeated multiple times, or combined with other techniques described herein, such that two or more cinching fixation system are used to secure the tissue or bone to the implant or humerus at several locations relative to the longitudinal axis of the humerus.

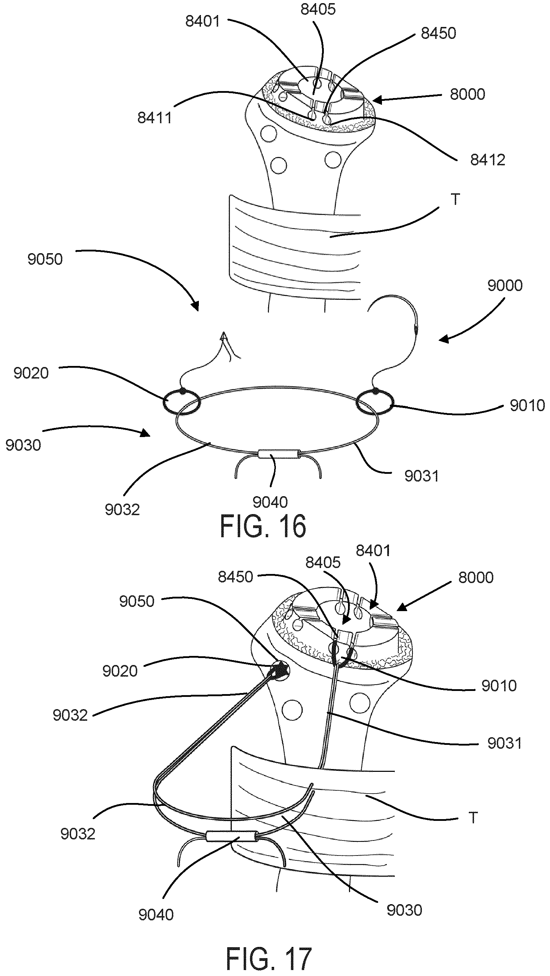

Although the implantable prosthesis described herein includes a stem, in some embodiments, the implantable prosthesis is a stemless implant 8000, such as a stemless shoulder prosthesis. FIGS. 16 and 17 generally depict shoulder joint total shoulder arthroplasty (TSA) techniques using a stemless implantable prosthesis 8000 and a cinching fixation system 9000 in accordance with aspects of the present disclosure. In some embodiments, the stemless implant 8000 includes a proximal end (not shown) and a distal end. The proximal end of the stemless implant is configured to mount onto or into a resected top surface of a humerus. The proximal end can include a contact surface that is in contact with a resected top surface of the humerus.

The distal end includes a joint mounting surface 8401 and a joint component retention mechanism 8405. The joint component retention mechanism 8405 includes a recess for receiving a protrusion of a joint component. In some embodiments, the joint component retention mechanism 8405 includes a female portion of a fastener. For example, the female portion can include female screw threads for receiving male screw threads of a joint component. In some embodiments, the retention mechanism 8405 includes a male portion of a fastener. For example, the male portion can include male screw threads for supporting female screw threads of a joint component.

The mounting surface 8401 defines a first mounting surface groove 8411, a second mounting surface groove 8412, and a mounting surface cleat 8450 defined between the first mounting surface groove 8411 and the second mounting surface groove 8412. It will be appreciated that additional sets of mounting surface grooves and mounting surface cleats can be provided on the joint mounting surface 8401.

In some embodiments, an implant system (or kit) includes the stemless implantable prosthesis 8000 and a cinching fixation system 9000. In some embodiments, the kit includes the implantable prosthesis 8000 and two to four cinching fixation systems 9000 to secure tissue T or bone at multiple locations to a bone or to the stemless implant. In some embodiments, the cinching fixation system 9000 includes a first mounting loop 9010, a second mounting loop 9020, a tensioning member 9030, a cinching member 9040, and an all-suture anchor or knot 9050. Examples of suitable tensioning members include Zimmer Biomet ZipLoop^{™} and Arthrex TightRope^{®}. The tensioning member includes a first tensioning end portion 9031 and a second tensioning end portion 9032. The first tensioning end portion 9031 is routed through the first mounting loop 9010 and routed through the cinching member 9040. The second tensioning end portion 9032 is routed through the second mounting loop 9020 and through the cinching member 9040. In some embodiments, the kit includes a piece of suture threaded through a needle and tied or coupled to the first mounting loop 9010. In some embodiments, the kit includes a piece of suture secured to the all-suture anchor or knot 9050 and secured to the second mounting loop 9020.

In some embodiments, not part of the present invention, a method of securing the tissue T to the bone includes passing the first mounting loop 9010' through the tissue T and securing the first mounting loop 9010 to the cleat 8450 of the stemless implantable prosthesis 8000. In some embodiments, the method includes inserting the all-suture anchor or knot 9050 into a bone tunnel formed into the humerus at the bicipital groove or any other suitable location. In use, the all-suture anchor or knot 9050 is expanded such that the all-suture anchor or knot 9050, together with a portion of the second mounting loop 9020 is secured within the bone tunnel. Although shown as including an all-suture anchor, in other embodiments, any suitable bone anchor (e.g., a bone screw or an expandable bone anchor) can be used. The method can further include pulling the first tensioning end portion 9031 and the second tensioning end portion 9032 of the tensioning member 9030 in a generally anterior direction to remove slack and to pull the first mounting loop 9010 to a side edge of the humerus. A joint component is then mounted to the implantable prosthesis such that the first mounting loop 9010 is secured between the joint component and the proximal end surface of the humerus.

The method includes pulling at least one tensioning end of the cinching fixation system 9000 through the cinching member 9040 to apply tension around the portion of tissue against the humerus. The tissue may be the subscapularis. The bone tunnel may be drilled through a portion of the humerus from a portion of the bicipital groove to the bore.

Although the cinching fixation system 9000 is shown and described as including two mounting loops, one of which (9010) is coupled to a needle and the other of which (9020) is coupled to an all-suture anchor (9050), in other embodiments, any of the kits or methods described herein can use a cinching fixation system having any suitable configuration of mounting loops, needles, and/or anchors. For example, a cinching fixation system can include three or more mounting loops to facilitate the fixation methods described herein.

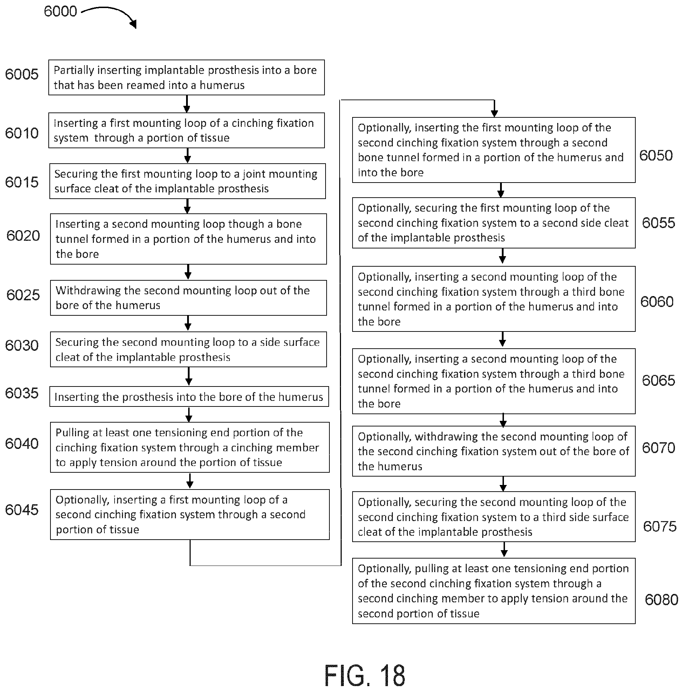

FIG. 18 is a flow chart showing a method 6000 of performing joint total shoulder arthroplasty including securing a portion of the subscapularis to the humerus using a stemmed humeral implant with cleat retention structures. The method includes at 6005 partially inserting an implantable prosthesis into a bore that has been reamed into a humerus. The implantable prosthesis includes a stem and a stem head coupled to the stem. The stem head includes a side surface and a joint mounting portion, the joint mounting portion being separate from the side surface. The joint mounting portion includes a joint mounting surface cleat.

The method includes at 6010 inserting a first mounting loop through the portion of tissue. The first mounting loop is coupled to a cinching fixation system. The method includes at 6015 securing the first mounting loop to the joint mounting surface cleat. In some embodiments, the method includes at 6020 inserting a second mounting loop through a bone tunnel drilled through a portion of the humerus and into the bore of the humerus. The second mounting loop is coupled to the cinching fixation system. The method includes at 6025 withdrawing the second mounting loop out of the bore of the humerus. The method includes at 6030 securing the second mounting loop to a side surface cleat of the side surface. The method includes at 6035 inserting the implantable prosthesis into the bore of the humerus. The method includes at 6040 pulling at least one tensioning end of the cinching fixation system through a cinching member to apply tension around the portion of the tissue against the humerus.

The portion of tissue is a first portion of tissue, the bone tunnel is a first bone tunnel, the side surface is a first side surface, the side surface cleat is a first side surface cleat, the cinching fixation system is a first cinching fixation system, the stem head including a second side surface, the second side surface includes a second side surface cleat. Optionally, the method includes at 6045 inserting a first mounting loop of a second cinching fixation system through a second portion of the tissue, the first mounting loop of the second cinching fixation system being coupled to the second cinching fixation system. Optionally, the method includes at 6050 inserting the first mounting loop of the second cinching fixation system through a second bone tunnel drilled through a portion of the humerus and into the bore of the humerus. Optionally, the method includes at 6055 securing the first mounting loop of the second cinching fixation system to second side surface cleat.

In some embodiments, the first side surface includes a third side surface cleat. Optionally, the method includes at 6060 inserting a second mounting loop of the second cinching fixation system through a third bone tunnel drilled through a portion of the humerus and into the bore of the humerus. The second mounting loop of the second cinching fixation system is coupled to the second cinching fixation system. Optionally, the method includes at 6065 withdrawing the second mounting loop of the second cinching fixation system out of the bore of the humerus. Optionally, the method includes at 6070 securing the second mounting loop of the second cinching fixation system to the third side surface cleat. Optionally, the method includes at 6070 securing the second mounting loop of the second cinching fixation system to the third side surface cleat. In some embodiments, the tissue is the subscapularis. Optionally, the method includes at 6075 pulling at least one tensioning end of the second cinching fixation system through a cinching member of the second cinching fixation system to apply tension around the second portion of the tissue against the humerus.

The bone tunnel may be drilled through a portion of the humerus from a portion of the bicipital groove to the bore. In some embodiments, the first bone tunnel is drilled through a portion of the humerus from a first portion of the bicipital groove into the bore, the second bone tunnel is drilled through a portion of the humerus medial to the lesser tuberosity and into the bore, and the third bone tunnel is drilled through a portion of the humerus from a second portion of the bicipital groove into the bore.

In some embodiments, not part of the present invention, a method of securing a portion of tissue or bone fragments to a bone or a prosthesis implant includes aligning the prosthesis implant with the bone. The prosthesis implant can be any of the implants described herein (e.g., the implant 1000, 3000 or 4000, which includes a stem, or the stemless implant 8000) and includes a joint mounting portion, a first cleat and a second cleat. The joint mounting portion including at least one of the first cleat or the second cleat. In some embodiments, the aligning can include maintaining the prosthesis implant at a position and/or orientation adjacent the implant site. For example, in some embodiments, the implantable prosthesis includes a stem and the aligning includes partially inserting the stem into a bore within the bone. In other embodiments, the aligning can include supporting the implant at the desired position and/or orientation using a tool, template or support member. In yet other embodiments, the implantable prosthesis is a stemless implant and the aligning includes placing a contact surface of the stemless implant into contact with a surface of the bone (e.g., a resected surface).

A first mounting loop is inserted through the portion of the tissue. The first (and a second) mounting loop can be any of the mounting loops described herein. The first mounting loop can be coupled to a suture needle and can be inserted through the portion of the tissue using the needle, as described above. The first mounting loop and a second mounting loop are each coupled to a cinching fixation system of the types shown and described herein.

The first mounting loop is secured to the first cleat. In some embodiments, the first mounting loop (and optionally, a portion of the cinching fixation system) can be routed through a bone tunnel, through a bore produced within the bone and then secured to the first cleat. In this manner, the suture / cinching fixation system is securely within the bone structure for retention purposes (see, e.g., FIG. 13). In other embodiments, such as procedures involving a stemless implant, the first mounting loop is secured to the first cleat (i.e., on the joint mounting portion) without being passed through a bone tunnel or bore (see, e.g., FIG. 17).

The second mounting loop is secured to the second cleat or a portion of the bone. In some embodiments, the second mounting loop (and optionally, a portion of the cinching fixation system) can be routed through a bone tunnel, through a bore produced within the bone and then secured to the second cleat. In this manner, the suture / cinching fixation system is securely within the bone structure for retention purposes (see, e.g., FIGS. 13 and 14). In other embodiments, the second mounting loop is secured to the second cleat without being passed through a bone tunnel or bore (see, e.g., FIG. 15). In yet other embodiments, such as procedures involving a stemless implant, the second mounting loop is not secured to the implant, but is instead secured to a portion of the bone (see, e.g., FIG. 17). In such embodiments, the second mounting loop can be secured to the bone using any suitable bone anchor system.

The prosthesis implant is coupled to the bone. Such coupling can include fully inserting a stem of the implant into a bore produced within the bone. Where the implant is a stemless implant, the coupling can include otherwise fixing the implant to a surface of the bone (e.g., via bone screws, bone cement or any other fixation mechanism. In some embodiments, one or more suture grooves (not shown) can be defined in the resected surface of the bone to allow portions of a mounting loop or the cinching fixation system to pass therethrough.

The method then includes pulling at least one tensioning end of the cinching fixation system to apply tension around the portion of tissue against the bone.

Although the methods described herein identify cleats on a particular surface that the mounting loops are mounting onto, it will be understood by one skilled in the art that any suitable cleat on the stemmed humeral implant can be used depending on the size of the mounting loop in relation a location of the tissue to be secured and/or the location of the bone tunnels. Additionally it will be understood by one skilled in the art that a portion of the humeral head can be resected and a bore reamed into the humerus prior to or in conjunction with the methods described herein. Furthermore, it will be understood by one skilled in the art that one or more the bone tunnels may be formed prior to or in conjunction with the methods described herein. For example, one or more of the bone tunnels may be formed by pre-drilling. Alternatively, the one or more tunnels may be formed by pressing firmly with a needle through the humerus as one of the mounting loops of the cinching fixation system is being passed through the humerus and into the bore.

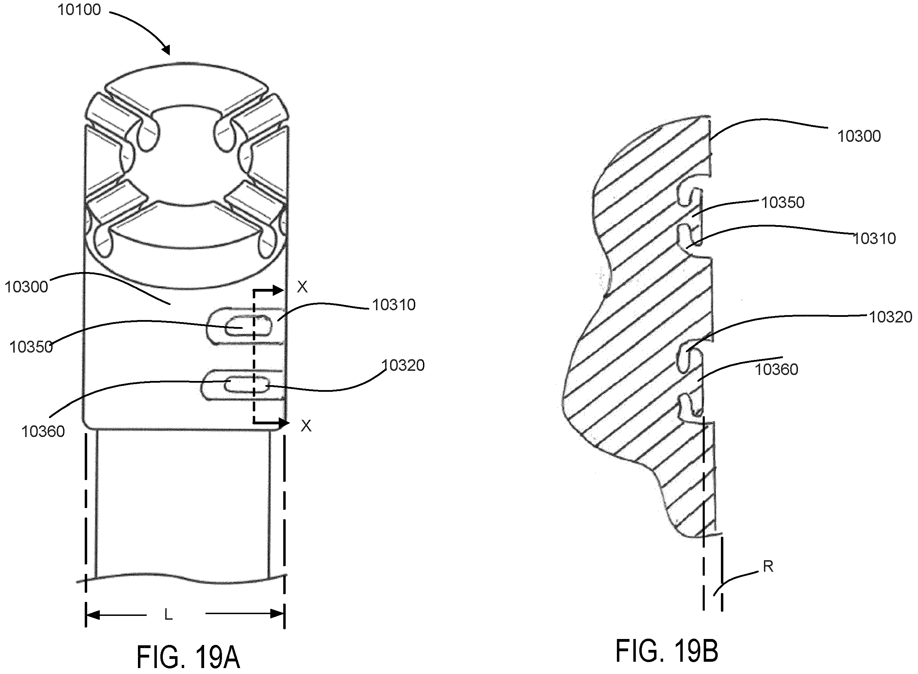

Although the implant 1000 is shown above as including a set of grooves (e.g., the grooves 1210 and 1220) that extend fully along the length of a side surface to define one or more cleats (e.g., the cleat 1250), in other embodiments, any of the implants described herein can include one or more grooves that extend only partially along the length of a side surface. Said another way, in some embodiments, a groove or recess have a length less than a length of the side surface. This arrangement can limit any surface disruption that may be caused by the inclusion of the grooves. Such an arrangement can also impact less of the surface area of the side surface, which may be more advantageous during insertion of the implant into a bore within a bone. For example, FIGS. 19A and 19B show a second side surface 10300 of stem head 10100 of an implant according to an embodiment. The stem head 10100 (and implant) can be similar to and include any of the features described above for the stem head 1100 and the implant 1000. Moreover, although FIGS. 19A and 19B show the second side surface 10300 of the stem head 10100, in other embodiments the grooves and cleats described can be included on any suitable surface of any implant described herein.

As shown, the second side surface 10300 defines a first groove 10310 and a second groove 10320. The stem head 10100 includes a first cleat 10350 and a second cleat 10360. The first cleat 10350 extends within the first groove 10310 and the second cleat 10360 extends within the second groove 10320. Thus, the first cleat 10350 is bounded (or surrounded) by the first groove 10310 and the second cleat 10360 is bounded (or surrounded) by the second groove 10210. The first groove 10310 and the second groove 10320 are each recesses that do not extend the full length L of the second side surface 10300. Rather, the first groove 10310 and the second groove 10320 surround the cleats (or form a pocket within which the cleats are disposed). As shown in FIG. 19B, the outer surface of the first cleat 10350 and the second cleat 10360 are recessed within the first groove 10310 and the second groove 10320, respectively, by a distance R.

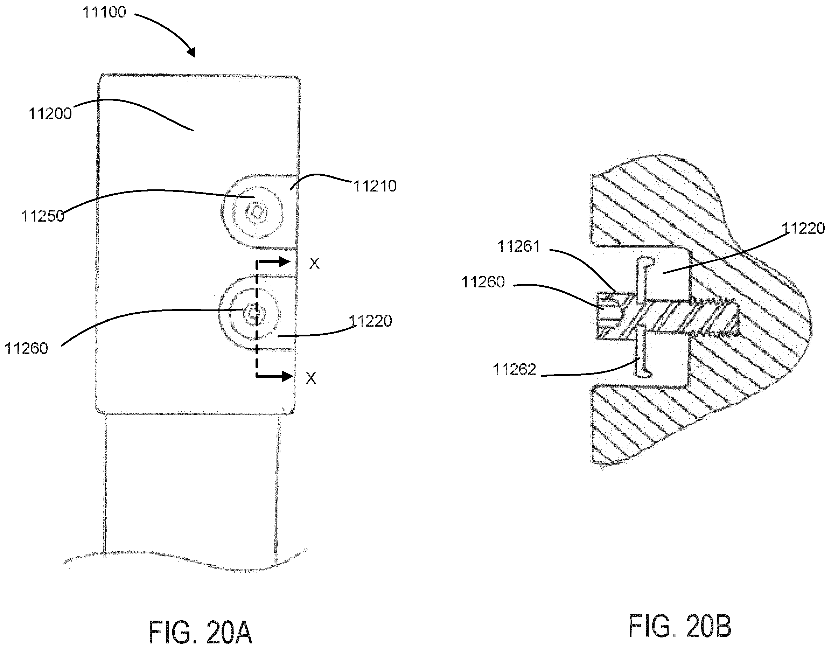

Although the implant 1000 is shown above as including cleats that are monolithically formed with the stem head 1100, in other embodiments, any of the implants described herein can include one or more cleats that are constructed separately from and the coupled to the stem head. Similarly stated, although the implant 1000 is shown as including cleats that are produced by defining grooves surrounding a portion of material from which the implant is constructed, in other embodiments, any of the implants described herein can include one or more cleats that are coupled within a pocket or recess of the implant. For example, FIGS. 20A and 20B show a first side surface 11200 of stem head 11100 of an implant according to an embodiment. The stem head 11100 (and implant) can be similar to and include any of the features described above for the stem head 1100 and the implant 1000. Moreover, although FIGS. 20A and 20B show the second side surface 11200 of the stem head 11100, in other embodiments the grooves and cleats described can be included on any suitable surface of any implant described herein.

As shown, the first side surface 11200 defines a first groove (or recess) 11210 and a second groove (or recess) 11220. The stem head 11100 includes a first cleat 11250 and a second cleat 11260. The first cleat 11250 extends within the first groove 11210 and the second cleat 112260 extends within the second groove 11220. Thus, the first cleat 11250 is bounded (or surrounded) by the first groove 11210 and the second cleat 11260 is bounded (or surrounded) by the second groove 11210. Similar to the stem head 10100 described above, the first groove 11210 and the second groove 11220 are each recesses that do not extend the full length of the second side surface 10300. Rather, the first groove 11210 and the second groove 11220 surround the cleats (or form a pocket within which the cleats are disposed).

As shown in FIG. 20B, the second cleat 11260 is a separate structure that is coupled to the stem head 11100 within the second groove 11220. In this embodiment, the second cleat 11260 (and also the first cleat 11250) includes a fastener 11261 (e.g., a cap screw) and a captive washer 11262. The fastener 11261 is coupled to the stem head 11100 (e.g., via threads, a press fit, an adhesive joint, or any other suitable mechanism). The captive washer 11262 produces the desired opening into the main portion of the groove 11220, as well as any desired undercut to improve retention of the suture within the groove 11220.

While various embodiments have been described above, it should be understood that they have been presented by way of example only, and not limitation. For example, although the implantable prosthesis is shown and described as being capable of being coupled to a joint component, such as a humeral head prosthesis for conventional total arthroplasty procedures, in some embodiments, the implantable prosthesis may include or may be coupled to a humeral cap for interfacing with a glenoid sphere for reverse shoulder arthroplasty procedures. Where methods and/or schematics described above indicate certain events and/or flow patterns occurring in certain order, the ordering of certain events and/or operations may be modified. Additionally, while the implants and methods described herein are used to repair a humerus and secure tissue back to the humerus, the implants and methods of the present disclosure can be used to secure tissue or additional implants to the humerus or to any other bone. For example, the implantable prosthesis may be used to secure tissue to a femur in a hip replacement procedure. Moreover, although the implants and methods described herein identify implantable prostheses as including a stem insertable into a bone, the implantable prosthesis with cleats may be secured to an external surface of a bone such that tissues and/or bone fragments from fracture can be secured back to the bone via the implantable prosthesis.

Any of the methods described herein can be performed by any of the implantable prosthesis described herein.

## Claims

1. An implantable prosthesis (3000), comprising:
a stem (3050) extending along a longitudinal axis (LA) from a proximal end portion (3051) of the stem to a distal end portion (3052) of the stem;
a stem head (3100) coupled to the proximal end portion (3051) of the stem, the stem head configured to be coupled to a joint component, the stem head (3100) including a first side surface (3200), a second side surface (3300),
**characterised by** the stem head (3100) further comprising:
a passageway sidewall (3700), the stem head defining a passageway (3600) extending through the stem head (3100) from the first side surface (3200) to the second side surface (3300), the passageway sidewall (3700) defining at least a portion of a boundary of the passageway (3600);
at least a portion of the passageway (3600) intersects the longitudinal axis (LA) of the stem (3050); and
the passageway sidewall (3700) includes a passageway cleat (3750) and defines a first passageway groove (3710) and a second passageway groove (3720), the passageway cleat (3750) extending between the first passageway groove (3710) and the second passageway groove (3720).

2. The implantable prosthesis (3000) of claim 1, wherein:
the passageway sidewall (3700) is a first passageway sidewall;
the passageway cleat (3750) is a first passageway cleat;
the portion of the boundary of the passageway (3600) is a first portion;
the stem head (3100) includes a second passageway sidewall (3800) defining at least a second portion of the boundary of the passageway (3600), the second passageway sidewall (3800) faces the first passageway sidewall (3700);
the second passageway sidewall (3800) defines a third passageway groove (3730) and a fourth passageway groove (3810); and
the second passageway sidewall (3800) includes a second passageway cleat (3760), the second passageway cleat extending between the third passageway groove (3730) and the fourth passageway groove (3810).

3. The implantable prosthesis (3000) of claim 1 or 2, wherein:
the first side surface (3200) is an anterior side surface; and
the second side surface (3300) is a posterior side surface.

4. The implantable prosthesis (3000) of any of claims 1 to 3, wherein:
the passageway cleat (3750) is a first passageway cleat;
the passageway sidewall (3700) defines a third passageway groove (3730) and includes a second passageway cleat (3760); and
the second passageway cleat (3760) extends between the second passageway groove (3720) and the third passageway groove (3730).

5. The implantable prosthesis (3000) of any of claims 1 to 4, wherein:
the passageway sidewall (3700) is a first passageway sidewall and the passageway cleat (3750) is a first passageway cleat;
the passageway (3600) is defined, at least in part, by a second passageway sidewall (3800), the second passageway sidewall faces the first passageway sidewall (3700);
the second passageway sidewall (3800) defines a third passageway groove (3730) and a fourth passageway groove (3810); and
the second passageway sidewall (3800) includes a second passageway cleat (3760), the second passageway cleat extending between the third passageway groove (3730) and the fourth passageway groove (3810).

6. The implantable prosthesis (3000) of claim 1, wherein:
the first surface (3200) is configured to be coupled to a surface of a bone; and
the second surface (3300) is opposite the first surface (3200), the second surface configured to be coupled to a joint component,
the second surface (3300) defining the first passageway groove (3710) and the second passageway groove (3720), the second surface (3300) including the passageway cleat (3750) extending between the first groove (3710) and the second groove (3720).

7. The implantable prosthesis (3000) of claim 6, wherein the second
surface (3300) defines a mounting recess configured to receive a mounting protrusion of the joint component.

8. The implantable prosthesis (3000) of claim 6 or 7, wherein the second
surface (3300) includes a mounting protrusion configured to mate with a mounting recess of the joint component.

9. A kit, comprising:
the implantable prosthesis (3000) of any preceding claim; and
a cinching fixation system (5000) including a mounting loop (5010) configured to be secured to the cleat (1750, 3750).

10. The kit of claim 9, wherein the implantable prosthesis (3000) is an implantable prosthesis (3000) according to any of claims 6 to 8; and
wherein said mounting loop (5010) of the cinching fixation system (5000) is a first mounting loop (5010);
the cinching fixation system (5000) further includes a second mounting loop (5020),
the first mounting loop (5010) and the second mounting loop (5020) each coupled to an adjustable suture component (5030), the second mounting loop (5020) including a bone anchor configured to couple the second mounting loop (5020) to the bone.

## Patentansprüche

1. Implantierbare Prothese (3000), umfassend:
einen Schaft (3050), der sich entlang einer Längsachse (LA) von einem proximalen Endabschnitt (3051) des Schafts zu einem distalen Endabschnitt (3052) des Schafts erstreckt;
einen Schaftkopf (3100), der mit dem proximalen Endabschnitt (3051) des Schafts gekoppelt ist, wobei der Schaftkopf ausgebildet ist, um mit einer Gelenkkomponente gekoppelt zu sein, wobei der Schaftkopf (3100) eine erste Seitenoberfläche (3200), eine zweite Seitenoberfläche (3300) einschließt,
**dadurch gekennzeichnet, dass** der Schaftkopf (3100) weiter umfasst:
eine Durchgangsseitenwand (3700), wobei der Schaftkopf einen Durchgang (3600) definiert, der sich durch den Schaftkopf (3100) hindurch von der ersten Seitenoberfläche (3200) zur zweiten Seitenoberfläche (3300) erstreckt, wobei die Durchgangsseitenwand (3700) mindestens einen Abschnitt einer Begrenzung des Durchgangs (3600) definiert;
wobei mindestens ein Abschnitt des Durchgangs (3600) die Längsachse (LA) des Schafts (3050) schneidet; und
wobei die Durchgangsseitenwand (3700) eine Durchgangsklemme (3750) einschließt und eine erste Durchgangsnut (3710) und eine zweite Durchgangsnut (3720) definiert, wobei sich die Durchgangsklemme (3750) zwischen der ersten Durchgangsnut (3710) und der zweiten Durchgangsnut (3720) erstreckt.

2. Implantierbare Prothese (3000) nach Anspruch 1, wobei:
die Durchgangsseitenwand (3700) eine erste Durchgangsseitenwand ist;
die Durchgangsklemme (3750) eine erste Durchgangsklemme ist;
der Abschnitt der Begrenzung des Durchgangs (3600) ein erster Abschnitt ist;
der Schaftkopf (3100) eine zweite Durchgangsseitenwand (3800) einschließt, die mindestens einen zweiten Abschnitt der Begrenzung des Durchgangs (3600) definiert, wobei die zweite Durchgangsseitenwand (3800) der ersten Durchgangsseitenwand (3700) zugewandt ist;
die zweite Durchgangsseitenwand (3800) eine dritte Durchgangsnut (3730) und eine vierte Durchgangsnut (3810) definiert; und
die zweite Durchgangsseitenwand (3800) eine zweite Durchgangsklemme (3760) einschließt, wobei sich die zweite Durchgangsklemme zwischen der dritten Durchgangsnut (3730) und der vierten Durchgangsnut (3810) erstreckt.

3. Implantierbare Prothese (3000) nach Anspruch 1 oder 2, wobei:
die erste Seitenoberfläche (3200) eine vordere Seitenoberfläche ist; und
die zweite Seitenoberfläche (3300) eine hintere Seitenoberfläche ist.

4. Implantierbare Prothese (3000) nach einem der Ansprüche 1 bis 3, wobei:
die Durchgangsklemme (3750) eine erste Durchgangsklemme ist;
die Durchgangsseitenwand (3700) eine dritte Durchgangsnut (3730) definiert und eine zweite Durchgangsklemme (3760) einschließt; und
die zweite Durchgangsklemme (3760) sich zwischen der zweiten Durchgangsnut (3720) und der dritten Durchgangsnut (3730) erstreckt.

5. Implantierbare Prothese (3000) nach einem der Ansprüche 1 bis 4, wobei:
die Durchgangsseitenwand (3700) eine erste Durchgangsseitenwand ist, und die Durchgangsklemme (3750) eine erste Durchgangsklemme ist;
der Durchgang (3600) mindestens teilweise durch eine zweite Durchgangsseitenwand (3800) definiert ist, wobei die zweite Durchgangsseitenwand der ersten Durchgangsseitenwand (3700) zugewandt ist;
die zweite Durchgangsseitenwand (3800) eine dritte Durchgangsnut (3730) und eine vierte Durchgangsnut (3810) definiert; und
die zweite Durchgangsseitenwand (3800) eine zweite Durchgangsklemme (3760) einschließt, wobei sich die zweite Durchgangsklemme zwischen der dritten Durchgangsnut (3730) und der vierten Durchgangsnut (3810) erstreckt.

6. Implantierbare Prothese (3000) nach Anspruch 1, wobei:
die erste Oberfläche (3200) ausgebildet ist, um mit einer Oberfläche eines Knochens gekoppelt zu sein; und
die zweite Oberfläche (3300) der ersten Oberfläche (3200) gegenüberliegt, wobei die zweite Oberfläche ausgebildet ist, um mit einer Gelenkkomponente gekoppelt zu sein,
die zweite Oberfläche (3300) die erste Durchgangsnut (3710) und die zweite Durchgangsnut (3720) definiert, die zweite Oberfläche (3300) die Durchgangsklemme (3750) einschließt, die sich zwischen der ersten Nut (3710) und der zweiten Nut (3720) erstreckt.

7. Implantierbare Prothese (3000) nach Anspruch 6, wobei die zweite Oberfläche (3300) eine Montagevertiefung definiert, die ausgebildet ist, um einen Montagevorsprung der Gelenkkomponente aufzunehmen.

8. Implantierbare Prothese (3000) nach Anspruch 6 oder 7, wobei die zweite Oberfläche (3300) einen Montagevorsprung einschließt, der ausgebildet ist, um mit einer Montagevertiefung der Gelenkkomponente zusammenzupassen.

9. Satz, umfassend:
die implantierbare Prothese (3000) nach einem vorstehenden Anspruch; und
ein Zuziehfixiersystem (5000), das eine Montageschlaufe (5010) einschließt, die ausgebildet ist, um an der Klemme (1750, 3750) befestigt zu werden.

10. Satz nach Anspruch 9, wobei die implantierbare Prothese (3000) eine implantierbare Prothese (3000) nach einem der Ansprüche 6 bis 8 ist; und
wobei die Montageschlaufe (5010) des Zuziehfixiersystems (5000) eine erste Montageschlaufe (5010) ist;
das Zuziehfixiersystem (5000) weiter eine zweite Montageschlaufe (5020) einschließt,
wobei die erste Montageschlaufe (5010) und die zweite Montageschlaufe (5020) jeweils an eine verstellbare Nahtkomponente (5030) gekoppelt sind, die zweite Montageschlaufe (5020) einen Knochenanker einschließt, der ausgebildet ist, um die zweite Montageschlaufe (5020) an den Knochen zu koppeln.

## Revendications

1. Prothèse implantable (3000), comprenant :
une tige (3050) s'étendant le long d'un axe longitudinal (LA) d'une portion d'extrémité proximale (3051) de la tige à une portion d'extrémité distale (3052) de la tige ;
une tête de tige (3100) couplée à la portion d'extrémité proximale (3051) de la tige, la tête de tige étant configurée pour être couplée à un composant d'articulation, la tête de tige (3100) incluant une première surface latérale (3200), une deuxième surface latérale (3300),
**caractérisée en ce que** la tête de tige (3100) comprend en outre :
une paroi latérale de passage (3700), la tête de tige définissant un passage (3600) s'étendant à travers la tête de tige (3100) de la première surface latérale (3200) à la deuxième surface latérale (3300), la paroi latérale de passage (3700) définissant au moins une portion d'une limite du passage (3600) ;
au moins une portion du passage (3600) croise l'axe longitudinal (LA) de la tige (3050) ; et
la paroi latérale de passage (3700) inclut un taquet de passage (3750) et définit une première rainure de passage (3710) et une deuxième rainure de passage (3720), le taquet de passage (3750) s'étendant entre la première rainure de passage (3710) et la deuxième rainure de passage (3720).

2. Prothèse implantable (3000) selon la revendication 1, dans laquelle :
la paroi latérale de passage (3700) est une première paroi latérale de passage ;
le taquet de passage (3750) est un premier taquet de passage ;
la portion de la limite du passage (3600) est une première portion ;
la tête de tige (3100) inclut une deuxième paroi latérale de passage (3800) définissant au moins une deuxième portion de la limite du passage (3600), la deuxième paroi latérale de passage (3800) faisant face à la première paroi latérale de passage (3700) ;
la deuxième paroi latérale de passage (3800) définit une troisième rainure de passage (3730) et une quatrième rainure de passage (3810) ; et
la deuxième paroi latérale de passage (3800) inclut un deuxième taquet de passage (3760), le deuxième taquet de passage s'étendant entre la troisième rainure de passage (3730) et la quatrième rainure de passage (3810).

3. Prothèse implantable (3000) selon la revendication 1 ou la revendication 2, dans laquelle :
la première surface latérale (3200) est une surface latérale antérieure ; et
la deuxième surface latérale (3300) est une surface latérale postérieure.

4. Prothèse implantable (3000) selon l'une quelconque des revendications 1 à 3, dans laquelle :
le taquet de passage (3750) est un premier taquet de passage ;
la paroi latérale de passage (3700) définit une troisième rainure de passage (3730) et inclut un deuxième taquet de passage (3760) ; et
le deuxième taquet de passage (3760) s'étend entre la deuxième rainure de passage (3720) et la troisième rainure de passage (3730).

5. Prothèse implantable (3000) selon l'une quelconque des revendications 1 à 4, dans laquelle :
la paroi latérale de passage (3700) est une première paroi latérale de passage et le taquet de passage (3750) est un premier taquet de passage ;
le passage (3600) est défini, au moins en partie, par une deuxième paroi latérale de passage (3800), la deuxième paroi latérale de passage faisant face à la première paroi latérale de passage (3700) ;
la deuxième paroi latérale de passage (3800) définit une troisième rainure de passage (3730) et une quatrième rainure de passage (3810) ; et
la deuxième paroi latérale de passage (3800) inclut un deuxième taquet de passage (3760), le deuxième taquet de passage s'étendant entre la troisième rainure de passage (3730) et la quatrième rainure de passage (3810).

6. Prothèse implantable (3000) selon la revendication 1, dans laquelle :
la première surface (3200) est configurée pour être couplée à une surface d'un os ; et
la deuxième surface (3300) est opposée à la première surface (3200), la deuxième surface étant configurée pour être couplée à un composant d'articulation,
la deuxième surface (3300) définissant la première rainure de passage (3710) et la deuxième rainure de passage (3720), la deuxième surface (3300) incluant le taquet de passage (3750) s'étendant entre la première rainure (3710) et la deuxième rainure (3720).

7. Prothèse implantable (3000) selon la revendication 6, dans laquelle la deuxième surface (3300) définit un évidement de montage configuré pour recevoir une saillie de montage du composant d'articulation.

8. Prothèse implantable (3000) selon la revendication 6 ou la revendication 7, dans laquelle la deuxième surface (3300) inclut une saillie de montage configurée pour s'accoupler à un évidement de montage du composant d'articulation.

9. Kit, comprenant :
la prothèse implantable (3000) selon une quelconque revendication précédente ; et
un système de fixation par serrage (5000) incluant une boucle de montage (5010) configurée pour être fixée au taquet (1750, 3750).

10. Kit selon la revendication 9, dans lequel la prothèse implantable (3000) est une prothèse implantable (3000) selon l'une quelconque des revendications 6 à 8 ; et
dans lequel ladite boucle de montage (5010) du système de fixation par serrage (5000) est une première boucle de montage (5010) ;
le système de fixation par serrage (5000) inclut en outre une deuxième boucle de montage (5020),
la première boucle de montage (5010) et la deuxième boucle de montage (5020) couplées chacune à un composant de suture réglable (5030), la deuxième boucle de montage (5020) incluant un ancrage osseux configuré pour coupler la deuxième boucle de montage (5020) à l'os.
